# EUROPEAN PATENT APPLICATION

(11) **EP 1 870 400 A1**
(43) Date of publication of application: **26.12.2007**
(21) Application number: 06115159.3
(22) Date of filing: 08.06.2006
(51) Int. Cl.: C07D 209/34, A61K 31/404, A61P 35/04

(54) **Salts and crystalline salt forms of an 2-indolinone derivative**

(71) Applicant: Boehringer Ingelheim Pharma GmbH & Co. KG, 55216 Ingelheim (DE)
(72) Inventor: Sieger, Peter, 88441 Mittelbiberach (DE); Rall, Werner, 88441 Mittelbiberach (DE); Linz, Günter, 88441 Mittelbiberach (DE); Mazurek, Jaroslaw, 2563 AV Den Haag (NL); Pop, Mihaela, 1060 NX, Amsterdam (NL)

(57) **Abstract**

The present invention relates to new salts and crystalline salt forms of an indolinone derivative which have valuable pharmacological properties, to a process for their manufacture, to pharmaceutical formulations containing them and to their use as medicament.

## Description

The present invention relates to new salts and crystalline salt forms of an indolinone derivative, namely of the compound 3-Z-[1-(4-(N-((4-methyl-piperazin-1-yl)-methylcarbonyl)-N-methyl-amino)-anilino)-1-phenyl-methylene] -6-methoxycarbonyl-2-indolinone, which have valuable pharmacological properties, to a process for their manufacture, to pharmaceutical formulations containing them and to their use as medicament.

### Background to the invention

The compound 3-Z-[1-(4-(N-((4-methyl-piperazin-1-yl)-methylcarbonyl)-N-methyl-amino)-anilino)-1-phenyl-methylene]-6-methoxycarbonyl-2-indolinone is known from WO 01/27081 and has the following chemical structure, depicted below as Formula (I)

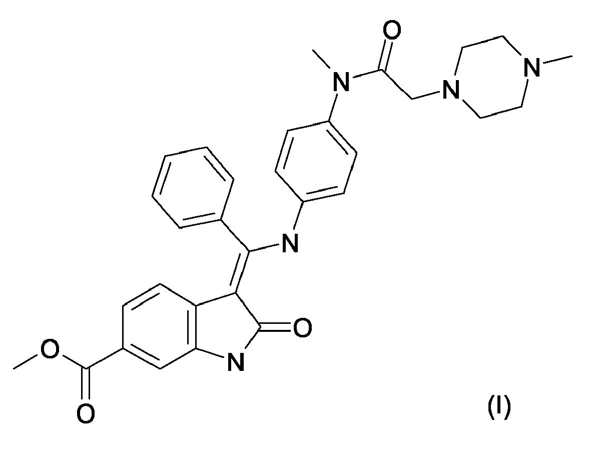

Furthermore, the monoethanesulfonate salt of this compound is known from WO 04/13099 in its crystalline hemihydrate form, as well as a process for its manufacture.

The above mentioned patent applications further disclose the use of this compound or its monoethanesulfonate salt for the preparation of pharmaceutical compositions intended especially for the treatment of diseases characterized by excessive or abnormal cell proliferation.

Furthermore, WO 04/17948 discloses the use of this compound for the preparation of pharmaceutical compositions for the treatment of immunologic diseases or pathological conditions involving an immunologic component.

### Summary of the invention

The aim of the present invention is to provide new salts and crystalline salt forms of the compound 3-Z-[1-(4-(N-((4-methyl-piperazin-1-yl)-methylcarbonyl)-N-methyl-amino)-anilino)-1-phenyl-methylene]-6-methoxycarbonyl-2-indolinone as such which are characterized by advantageous physicochemical properties.

Another object of the invention is to provide new salts and crystalline salt forms of the compound 3-Z-[1-(4-(N-((4-methyl-piperazin-1-yl)-methylcarbonyl)-N-methyl-amino)-anilino)-1-phenyl-methylene]-6-methoxycarbonyl-2-indolinone as such which are characterized by unexpected, new pharmacological properties.

### Detailed description of the invention

The invention relates to a process for the preparation of the new salts and crystalline salt forms of the compounds of formula (I), optionally in the form of the individual optical isomers, mixtures of the individual enantiomers or racemates, and optionally to the hydrates and/or solvates thereof.

### Process for the preparation of the salts

In the process according to the invention the free base of the compound of formula (I) is dissolved in a suitable solvent, such as 2,2,2-trifluoro-ethanol. The acid used for the crystallization is dissolved as well in a suitable solvent, such as 2,2,2-trifluoroethanol/water (50:50) or water (depending on the acid). The free base of the compound of formula (I) is then mixed with the acid at a predetermined base /acid molar ratio, which is selected from 1:1 or 2:1 depending on the acid. Then, the solvent is evaporated under reduced pressure. After evaporation of the solvent has occurred, a suitable crystallization solvent is added to the reaction mixture, and the reaction mixture is slowly heated up to 50°C. Suitable solvents for the crystallization are tetrahydrofurane, dichloromethane, water, methanol, n-butylacetate, 1,2-dimethoxyethane, 2,2,2-trifluoroethanol (TFE)/water = 8:2, acetone/ dimethylsulfoxide (DMSO) = 8:2, chloroform, acetonitrile, ethanol, 1-methyl-2-pyrrolidinone (NMP)/water = 80:20, propyl acetate, tert. butanol, 1,4-dioxane, ethyl acetate, propionitrile, di-isopropyl ether, tert. butylethyl ether, ethanol/water = 80:20, nitrobenzene, cyclohexanone, ethyl phenyl ether, 2 - nitropropane, tert. butylmethyl ether and isobutanol. After staying for about 30 minutes at 50°C, the reaction mixture is slowly cooled down to a suitable crystallization temperature, which is for example between 20°C or 3°C. The reaction mixture stays at this temperature until enough crystals are formed, which can then be collected, for example by filtration.

The process is illustrated by the following example of manufacturing process of the salts and crystalline salt forms, as can be done in parallel in 96 well assay plates (maximum volume of each well is about 200 µl).

Approximately 1 g of the free base of the compound of formula (I) is dissolved in 10 ml of 2,2,2-trifluoroethanol. The acids used to prepare the salts are dissolved in 2,2,2-trifluoroethanol/water (50:50) or just in water as far as the L-aspartic acid is concerned. Each well of the 96 well plates is loaded with the free base of the compound of formula (I) as dissolved in 2,2,2-trifluoroethanol and with the acid as dissolved in the mixture of 2,2,2-trifluoroethanol/water (50:50) or just in water as far as the L-aspartic acid is concerned, such that the molar ratio of the compound of formula (I) to the respective acid is set according to the information given in Table 1 under "ratio base/acid". The 96 well plates are then placed in a vacuum chamber (1 kPa) at room temperature for 24 h in order to evaporate the solvent. Afterwards, different solvents are added in each well according to the information given in Table 1 under "crystallization solvent", and the well plates are sealed and heated up to 50 °C at a heating rate of approx. 5 °C/min. The plate stays then for an additional 30 minutes at 50 °C. Afterwards, the plate is cooled at a cooling rate of 5°C/h to a final temperature of 3 or 20 °C according to the information given in Table 1 under "T_{final}[°C]". At this temperature, the plates remain for a holding time of 24 h. The plates are then opened and the solids are collected by filtration.

**Table 1: Conditions for the preparation of the different salts of the compound 3-Z-[1-(4-(N-((4-methyl-piperazin-1-yl)-methylcarbonyl)-N-methyl-amino)-anilino)-1-phenyl-methylene]-6-methoxycarbonyl-2-indolinone**

| **salt form abbreviation** | **salt form full name** | **used acid** | **ratio base/acid** | **crystallization solvent** | **T_{final} [°C]** |
|---|---|---|---|---|---|
| HCl1 | chloride, form I | hydrochloric acid | 1:1 | tetrahydrofurane | 20 |
| HCl2 | chloride, form II | hydrochloric acid | 1:1 | dichloromethane | 3 |
| HBr2 | bromide, form II | hydrobromic acid | 1:1 | water | 20 |
| HBr7 | bromide, form VII | hydrobromic acid | 1:1 | methanol | 3 |
| HBr8 | bromide, form VIII | hydrobromic acid | 1:1 | n-butylacetate | 20 |
| Pho1 | phosphate, form I | phosphoric acid | 1:1 | 1,2-dimethoxyethane | 20 |
| Pho2 | phosphate, form II | phosphoric acid | 1:1 | TFE*/water = 8:2 | 3 |
| Sul1 | sulfate, form I | sulfuric acid | 1:1 | acetone/DMSO*=8:2 | 3 |
| Sul5 | sulfate, form V | sulfuric acid | 1:1 | chloroform | 3 |
| Sul6 | sulphate, form VI | sulfuric acid | 2:1 | 1,2-dimethoxyethane | 20 |
| Sul7 | sulphate, form VII | sulfuric acid | 2:1 | acetonitrile | 3 |
| Mes1 | mesylate, form I | methanesulfonic acid | 1:1 | ethanol | 20 |
| Eds1 | edisylate, form I | ethanedisulfonic acid | 1:1 | methanol | 3 |
| Eds2 | edisylate, form II | ethanedisulfonic acid | 1:1 | 1,2-dimethoxyethane | 3 |
| Eds3 | edisylate, form III | ethanedisulfonic acid | 1:1 | chloroform | 20 |
| Eds5 | edisylate, form V | ethanedisulfonic acid | 1:1 | NMP*/water=80:20 | 20 |
| Eds6 | edisylate, form VI | ethanedisulfonic acid | 1:1 | propyl acetate | 20 |
| Eds7 | edisylate, form VII | ethanedisulfonic acid | 2:1 | ethanol | 3 |
| Ise1 | isethionate, form I | isethionic acid or 2-hydroxyethanes ulfonic acid | 1:1 | tert. butanol | 20 |
| lse2 | isethionate, form II | isethionic acid or 2-hydroxyethanes ulfonic acid | 1:1 | 1,4-dioxane | 20 |
| Ise4 | isethionate, form IV | isethionic acid or 2-hydroxyethanesulfonic acid | 1:1 | ethyl acetate | 20 |
| Ise5 | isethionate, form V | isethionic acid or 2-hydroxyethanes ulfonic acid | 1:1 | propionitrile | 20 |
| Bes1 | besylate, form I | benzenesulfonic acid | 1:1 | methanol | 20 |
| Bes3 | besylate, form III | benzenesulfonic acid | 1:1 | ethyl acetate | 20 |
| Bes5 | besylate, form V | benzenesulfonic acid | 1:1 | tert. butanol | 20 |
| Tos1 | tosylate, form I | p-toluenesulfonic acid | 1:1 | 1,2-dimethoxyethane | 5 |
| Tos2 | tosylate, form II | p-toluenesulfonic acid | 1:1 | water | 3 |
| Cas2 | camphorsulf onate, form II | camphor-10-sulfonic acid | 1:1 | propionitrile | 3 |
| Cas3 | camphorsulf onate, form III | camphor-10-sulfonic acid | 1:1 | ethyl acetate | 20 |
| Cas5 | onate, form V | camphor-10-sulfonic acid | 1:1 | propionitrile | 20 |
| Cas12 | camphorsulf onate, form XII | camphor-10-sulfonic acid | 1:1 | 1,2-dimethoxyethane | 20 |
| Nds3 | naphthalene-1,5-disulfonate, form III | naphthalene-1,5-disulfonic acid | 1:1 | di-isopropyl ether | 3 |
| Nds4 | naphthalene-1,5-disulfonate, form IV | naphthalene-1,5-disulfonic acid | 1:1 | methanol | 20 |
| Nds5 | naphthalene-1,5-disulfonate, form V | naphthalene-1,5-disulfonic acid | 1:1 | propionitrile | 20 |
| Cit1 | citrate, form I | citric acid | 1:1 | 1,4-dioxane | 3 |
| Cit2 | citrate, form II | citric acid | 1:1 | methanol | 20 |
| D-Tar1 | D-tartrate, form I | D-tartaric acid | 1:1 | ethanol | 3 |
| L-Tar1 | L-tartrate, form I | L-tartaric acid | 1:1 | tert. butylethyl ether | 20 |
| D-Tar2 | D-tartrate, form II | D-tartaric acid | 2:1 | propionitrile | 3 |
| L-Tar2 | L-tartrate, form II | L-tartaric acid | 2:1 | ethyl acetate | 20 |
| Fum1 | fumarate, form I | fumaric acid | 1:1 | ethanol/water=80:20 | 3 |
| Fum3 | fumarate, form III | fumaric acid | 2:1 | methanol | 3 |
| Mae1 | maleate, form I | maleic acid | 1:1 | propionitrile | 20 |
| L-Lac1 | L-lactate, form I | L-lactic acid | 1:1 | propyl acetate | 20 |
| Glc1 | glucolate, form I | glycolic acid | 1:1 | propionitrile | 3 |
| Gly1 | glycinate, form I | glycine | 1:1 | nitrobenzene | 3 |
| L-Mal1 | L-malate, form I | L-malic acid | 1:1 | propionitrile | 3 |
| D-Mal1 | D-malate, form I | D-malic acid | 1:1 | propionitrile | 3 |
| L-Mal2 | L-malate, form II | L-malic acid | 1:1 | dichloromethane | 3 |
| L-Mal3 | L-malate, form III | L-malic acid | 1:1 | propyl acetate | 20 |
| L-Mal4 | L-malate, form IV | L-malic acid | 2:1 | cyclohexanone | 20 |
| D-Mal4 | D-malate, form IV | D-malic acid | 2:1 | cyclohexanone | 20 |
| Mao1 | malonate, form I | malonic acid | 1:1 | methanol | 20 |
| Mao2 | malonate, form II | malonic acid | 1:1 | propyl acetate | 3 |
| Mao3 | malonate, form III | malonic acid | 2:1 | 1,4-dioxane | 3 |
| Mao4 | malonate, form IV | malonic acid | 2:1 | ethyl acetate | 20 |
| Mao5 | malonate, form V | malonic acid | 2:1 | water | 20 |
| Mao6 | malonate, form VI | malonic acid | 2:1 | methanol | 3 |
| Suc1 | succinate, form I | succinic acid | 1:1 | methanol | 3 |
| Suc2 | succinate, form II | succinic acid | 1:1 | ethyl phenyl ether | 20 |
| Suc3 | succinate, form III | succinic acid | 1:1 | acetone/DMSO*=8:2 | 3 |
| Oxa3 | oxalate, form III | oxalic acid | 1:1 | 1,2-dimethoxyethane | 20 |
| Oxa5 | oxalate, form V | oxalic acid | 1:1 | NMP*/water=80:20 | 20 |
| Oxa6 | oxalate, form VI | oxalic acid | 2:1 | propionitrile | 3 |
| Gen1 | 2,5-dihydroxybenzoate form I | gentisic acid or 2,5-dihydroxybenzoic acid | 1:1 | 2 - nitropropane | 3 |
| Gen11 | 2,5-dihydroxybe nzoate form Xl | gentisic acid or 2,5-dihydroxybenzoic acid | 1:1 | 1,2-dimethoxyethane | 20 |
| Cam2 | camphorate, form II | camphoric acide | 1:1 | tert. butylmethyl ether | 20 |
| Cam3 | camphorate, form III | camphoric acide | 1:1 | propyl acetate | 3 |
| Ben2 | benzoate, form II | benzoic acid | 1:1 | acetone/DMSO*=8:2 | 3 |
| Ben3 | benzoate, form III | benzoic acid | 1:1 | propyl acetate | 3 |
| Man1 | mandelate, form I | S-(+)-mandelic acid | 1:1 | methanol | 3 |
| Sac2 | saccharinate, form II | saccharine | 1:1 | propyl acetate | 3 |
| Sac3 | saccharinate, form III | saccharine | 1:1 | 1,2-dimethoxyethane | 3 |
| Sac5 | saccharinate, form V | saccharine | 1:1 | water/DMSO*=8:2 | 3 |
| Sal1 | salicylate, form I | salicylic acid | 1:1 | water | 3 |
| Sal2 | salicylate, form II | salicylic acid | 1:1 | 1,2-dimethoxyethane | 20 |
| L-Asp1 | L-aspartate, form I | L-aspartic acid | 1:1 | 1,2-dimethoxyethane | 3 |
| L-Asp2 | L-aspartate, form II | L-aspartic acid | 1:1 | nitrobenzene | 20 |
| Xin1 | xinafoate, form I | 1-hydroxy-2-naphthoic acid | 1:1 | isobutanol | 3 |
| Asc1 | ascorbate, form I | ascorbic acid | 1:1 | 1,4 - dioxane | 20 |
| Asc3 | ascorbate, form III | ascorbic acid | 1:1 | acetonitrile | 3 |
| Asc4 | ascorbate, form IV | ascorbic acid | 1:1 | methanol | 3 |
| Asc6 | ascorbate, form VI | ascorbic acid | 1:1 | ethyl acetate | 20 |
| Asc7 | ascorbate, form VII | ascorbic acid | 1:1 | cyclohexanone | 3 |

| | | | | | |
|---|---|---|---|---|---|
| * TFE = 2,2,2-trifluoroethanol * DMSO = dimethylsulfoxide * NMP = 1-methyl-2-pyrrolidinone | | | | | |

A preferred embodiment according to the invention thus relates to the preparation of the new salts and crystalline salt forms of the compound in accordance with formula (I) as specified hereinbefore.

In another embodiment, the invention relates to solutions containing the compound of formula (I) dissolved or suspended, preferably dissolved in a solvent. In a preferred embodiment, the solvent is an alcohol, preferably 2,2,2-trifluoro-ethanol.

In particular, the invention relates to specific crystalline forms of the compound of formula (I), which are discussed and characterized in detail below.

### Analytical methods for the characterization of the salts

The harvested crystals may be characterized by X-ray powder diffraction and thermal analysis (DSC and in some cases also TGA). If suitable single crystals grow, single crystal X-ray structure analysis may be performed. The following equipment was used to characterize the crystalline salts forms.

### X-ray powder diffraction (= XRPD)

XRPD patterns were obtained using a high throughput XRPD set-up. The plates were mounted on a Bruker GADDS diffractometer equipped with a Hi-Star area detector. The diffractometer was calibrated using Silver Behenate for the long d-spacings and corundum for the short d-spacings.

The data collection was carried out at room temperature using monochromatic CuKα radiation in the region of 2Θ between 1.5 and 41.5 °. The diffraction pattern of each well was collected with an exposure time of 3 - 4 minutes.

### Single crystal X-ray structure analysis

Suitable single crystals were selected and glued to a glass fibre, which is mounted on a X-ray diffraction goniometer. X-ray diffraction data were collected for the mounted crystals at a temperature of 233 K using a KappaCCD system and MoKα radiation generated by a FR590 X-ray generator (Bruker Nonius Delft, The Netherlands). Unit-cell parameters and crystal structure were determined and refined using the software package maXus (Mackay et al., 1997).

### Thermal analysis (DSC and TGA)

Melting properties were obtained from differential scanning calorimetry (= DSC) thermograms recorded on a DSC822e (Mettler-Toledo GmbH, Switzerland). The DSC822e was calibrated for temperature and enthalpy with a small piece of indium (Tfus = 156.6 °C, ΔHfus = 28.45 J/g). Samples were sealed in standard 40 µl aluminium pans and heated in the DSC from 25 to 300 °C with a heating rate of 20 °C/min. Dry nitrogen gas was used to purge the DSC equipment during measurements at a flow rate of 50 ml/min.

The mass loss due to solvent or water loss from the crystals was determined by thermo gravimetric analysis (= TGA). During heating of a sample in a TGA/SDTA851e (Mettler-Toledo GmbH, Switzerland) the weight of the sample was monitored resulting in a weight vs. temperature curve. The TGA/SDTA851e was calibrated for temperature with indium and aluminium. Samples were weighed in 100 µl corundum crucibles and heated in the TGA from 25 to 300 °C with a heating rate of 20 °C/min. Dry nitrogen gas was used for purging.

The results of the characterization of the new salt forms of the compound of formula (I) are shown below in Table 2 and Tables 3.1 to 3.20.

**Table 2: Thermal analysis, stoichiometry and single crystal data of the different salts of the compound 3-Z-[1-(4-(N-((4-methyl-piperazin-1-yl)-methylcarbonyl)-N-methyl-amino)-anilino)-1-phenyl-methylene]-6-methoxycarbonyl-2-indolinone**

| **salt form abbreviation** | **salt form full name** | **stoichiometry (base:counter-ion)** | **thermal analysis (T_{fus}*& LOD*)** | **single crystal data** |
|---|---|---|---|---|
| Bas1 | free base (= hydrated form) | not relevant | T_{fus}=253°C LOD = 1.5 % | see Tab. 3.1 |
| HCl1 | chloride, form I (= anhydrous form) | 1:1 1 | T_{fus}= 275 °C LOD = 0.2 % | |
| HCl2 | chloride, form II (= hydrated form) | 1:1 | T_{fus}= 275 °C LOD = 8.9 % | |
| HBr2 | bromide, form II (= hydrated form) | 1:1 | n.d. | see Tab 3.2 |
| HBr7 | bromide, form VII (= anhydrous form) | 1:1 | T_{fus} = 250 °C LOD = 0.7 % | |
| HBr8 | bromide, form VIII | 1:1 | T_{fus} = 260 °C LOD = n.d. | |
| Pho1 | phosphate, form I | 1:1 | T_{fus}= 270 °C LOD: n.d. | |
| Pho2 | phosphate, form II (= hydrated form) | 1:1 | T_{fus} = 270 °C LOD = 8.5 % | see Tab 3.3 |
| Sul1 | sulfate, form I (= hydrated form) | 1:1 | T_{fus} = 270 °C LOD = 5.5 % | |
| Sul5 | sulfate, form V | 1:1 | n.d. | |
| Sul6 | sulfate, form VI (= hydrated form) | 2:1 | T_{fus} = 245 °C LOD = 6.6 % | |
| Sul7 | sulfate, form VII (= hydrated form) | 2:1 | T_{fus} = 240 °C LOD = n.d. | |
| Mes1 | mesylate, form I (= hydrated form) | 1:1 | T_{fus} = 255 °C LOD = 1.5 % | see Tab. 3.4 |
| Eds1 | edisylate, form I | 1:1 or 2:1 | n.d. | |
| Eds2 | edisylate, form II | 1:1 or 2:1 | n.d. | |
| Eds3 | edisylate, form III (= hydrated form) | 1:1 or 2:1 | T _{fus}: n.d. LOD = 4.90 % | |
| Eds5 | edisylate, form V | 1:1 or 2:1 | n.d. | |
| Eds6 | edisylate, form VI | 1:1 or 2:1 | n.d. | |
| Eds7 | edisylate, form VII (= anhydrous form) | 1:1 or 2:1 | T_{fus}: ca. 300 °C LOD < 0.5 % | |
| Ise1 | isethionate, form I (= anhydrous form) | 1:1 | T_{fus} = 285 °C LOD = 0.3 % | see Tab. 3.5 |
| lse2 | isethionate, form II | 1:1 | n.d. | |
| lse4 | isethionate, form IV | 1:1 | n.d. | |
| Ise5 | isethionate, form V (= hydrated form) | 1:1 | n.d. | see Tab 3.6 |
| Bes1 | besylate, form I (= hydrated form) | 1:1 | n.d. | see Tab. 3.7 |
| Bes3 | besylate, form III (= anhydrous form) | 1:1 | T_{fus} = 258 °C LOD < 0.5 % | |
| Bes5 | besylate, form V (= anhydrous form) | 1:1 | T_{fus}=237°C LOD < 0.5 % | |
| Tos1 | tosylate, form I (= hydrated form) | 1:1 | T_{fus} = 250 °C LOD = 1.5 % | see Tab. 3.8 |
| Tos2 | tosylate, form II (= hydrated form) | 1:1 | n.d. | see Tab. 3.9 |
| Cas2 | camphorsulfonate, form II (= anhydrous form) | 1:1 | T_{fus} = 265 °C LOD<0.5% | |
| Cas3 | camphorsulfonate, form III (= anhydrous form) | 1:1 | n.d. | see Tab. 3.10 |
| Cas5 | camphorsulfonate, form V (= solvate) | 1:1 | T_{fus} = 268 LOD = n.d. | see Tab. 3.11 |
| Cas12 | camphorsulfonate, form XII (= solvate) | 1:1 | n.d. | see Tab. 3.12 |
| Nds3 | naphthalene-1,5-disulfonate, form III | 1:1 | T_{fus} = 93 °C LOD = n.d. | |
| Nds4 | naphthalene-1,5-disulfonate, form IV | 1:1 | n.d. | |
| Nds5 | naphthalene-1,5-disulfonate, form V (= hydrated form) | 1:1 | T_{fus}: ca. 150°C LOD = 2.0 % | |
| Cit1 | citrate, form I (= anhydrous form) | 1:1 or 2:1 | T_{fus} = 200 °C LOD = 0.2 % | |
| Cit2 | citrate, form II (= hydrated form) | 2:1 | T_{fus} = 220 °C LOD = 3.0 % | see Tab. 3.13 |
| D-Tar1 | D-tartrate, form I (= hydrated form) | 1:1 | T_{fus} = 240 °C LOD = 3.1 % | |
| L-Tar1 | L-tartrate, form I (= hydrated form) | 1:1 | T_{fus} = 240 °C LOD = 3.1 % | |
| D-Tar2 | D-tartrate, form II | 2:1 | T_{fus} = 244 °C LOD = 4.2 % | |
| L-Tar2 | L-tartrate, form II | 2:1 | T_{fus} = 245 °C LOD = 4.0 % | |
| Fum1 | fumarate, form I (= hydrated form) | 2:1 | T_{fus} = 265 °C LOD = 7.9 % | |
| Fum3 | fumarate, form III (= anhydrous form) | 2:1 | T_{fus} = 263 °C LOD < 0.5 % | |
| Mae1 | maleate, form I (= anhydrous form) | 2:1 | T_{fus} = 225 °C LOD = 0.6 % | |
| L-Lac1 | L-lactate, form I (= hydrated form) | 1:1 | T_{fus} = 220 °C LOD = 6.7 % | see Tab. 3.14 |
| Glc1 | glycolate, form I (= hydrated form) | 1:1 | T_{fus} = 230 °C LOD = 6.9 % | |
| Gly1 | glycinate, form I | 1:1 | n.d. | |
| L-Mal1 | L-malate, form I (= hydrated form) | 1:1 | T_{fus} = 215 °C LOD = 2.5 % | |
| D-Mal1 | D-malate, form I (= hydrated form) | 1:1 | T_{fus} = 215 °C LOD = 2.5 % | |
| L-Mal2 | L-malate, form II | 1:1 or 2:1 | n.d. | |
| L-Mal3 | L-malate, form III (= hydrated form) | 2:1 | n.d. | see Tab. 3.15 |
| L-Mal4 | L-malate, form lV | 2:1 | T_{fus}= 220 °C LOD: n.d. | |
| D-MaI4 | D-malate, form lV | 2:1 | T_{fus}= 220 °C LOD: n.d. | |
| Mao1 | malonate, form (= hydrated form) | 1:1 or 2:1 | T_{fus}: ca. 185 °C LOD = 4.3 % | |
| Mao2 | malonate, form II (= hydrated form) | 1:1 or 2:1 | T_{fus}: ca. 185 °C LOD = 7.4 % | |
| Mao3 | malonate, form III (= hydrated and/or solvated form) | 2 : 1 | T _{fus} = 251 °C LOD = 13.7 % | |
| Mao4 | malonate, form IV | 2:1 | T_{fus} = 252 °C LOD: n.d. | |
| Mao5 | malonate, form V | 2:1 | n.d. | |
| Mao6 | malonate, form VI | 2:1 | T_{fus} = 252 °C LOD: n.d. | |
| Suc1 | succinate, form I (= hydrated form) | 2:1 | T_{fus} = 139 °C LOD = 1.2 % | |
| Suc2 | succinate, form II (= hydrated form) | 2:1 | T_{fus}: ca. 220 °C LOD = 2.0 % | |
| Suc3 | succinate, form III (= hydrated form) | 2:1 | T_{fus}: ca. 210 °C LOD = 8.4 % | see Tab. 3.16 |
| Oxa3 | oxalate, form III (= hydrated form) | 1:1 or 2:1 | T_{fus}= 245 °C LOD = 5.7 % | |
| Oxa5 | oxalate, form V | 1:1 or 2:1 | n.d. | |
| Oxa6 | oxalate, form VI (= hydrated form) | 2:1 | T_{fus} = 244 °C LOD = 7.0 % | |
| Gen1 | 2,5-dihydroxybenzoate form I (= hydrated form) | 1:1 | T_{fus}: ca. 225 °C LOD = 2.3 % | |
| Gen11 | 2,5-dihydroxybenzoate form XI (= hydr. form) | 1:1 | n.d. | see Tab. 3.17 |
| Cam2 | camphorate, form II (= hydrated form) | 1:1 | T_{fus}: n.d. LOD: 1.9 %. | |
| Cam3 | camphorate, form III | 1:1 | T_{fus}: n.d. LOD: 0.5 % | |
| Ben2 | benzoate, form II (= hydrated form | 1:1 | T_{fus} = 175 °C LOD = 6.0 % | |
| Ben3 | benzoate, form III | 1:1 | n.d. | |
| Man1 | mandelate, form I (= hydrated form) | 1:1 | T_{fus} = 255 °C LOD = 2.6 | see Tab. 3.18 |
| Sac2 | saccharinate, form II (= hydrated form) | 1:1 | T_{fus} = 225 °C LOD = 2.6 % | |
| Sac3 | saccharinate, form III | 1:1 | n.d. | |
| Sac5 | saccharinate, form V (= hydrated form) | 1:1 | T_{fus} = 180 °C LOD = 2.8 % | |
| Sal1 | salicylate, form | 1:1 | T_{fus} = 248 °C LOD: n.d. | |
| Sal2 | salicylate, form II (= anhydrous form) | 1:1 | T_{fus} = 245 °C LOD: n.d. | see Tab. 3.19 |
| L-Asp1 | L-aspartate, form I (= hydrated form) | 2:1 | T_{fus} = 245 °C LOD = 6.8 % | |
| L-Asp2 | L-aspartate, form II | 2:1 | n.d. | |
| Xin1 | xinafoate, form I (= hydrated form) | 1:1 | T_{fus} = 164 °C LOD = 2.8 % | see Tab. 3.20 |
| Asc1 | ascorbate, form I | 1:1 | T_{fus} = 247 °C LOD = n.d. | |
| Asc3 | ascorbate, form III | 1:1 | T_{fus} = 234 °C LOD = n.d. | |
| Asc4 | ascorbate, form IV | 1:1 | T_{fus} = 236 °C LOD = n.d. | |
| Asc6 | ascorbate, form VI | 1:1 | n.d. | |
| Asc7 | ascorbate, form VII | 1:1 | n.d. | |

| | | | | |
|---|---|---|---|---|
| * T_{fus} = melting point *LOD = loss on drying up to the melting point n.d. = not determined | | | | |

**Table 3.1 Single crystal data and structure refinement of Bas1 = free base (= hemihydrate)**

| | |
|---|---|
| Empirical formula | C₃₁H₃₄N₅O₄ • 0.5 H₂O |
| Fw | 549.64 |
| T [K] | 293(2) K |
| λ[Å] | 0.71073 Å |
| Crystal system | Triclinic |
| Space group | P -1 |
| Unit cell dimensions | |
| a [Å] | 10.5430(3) |
| b [Å] | 10.9130(4) |
| c [Å] | 13.8380(6) |
| α[Å] | 78.147 (2) |
| β [°] | 89.713(3) |
| γ [°] | 67.834(4) |
| V [A³] | 1438.41(9) |
| Z | 2 |
| Dₘ [g/cm³] | 1.269 |
| F(000) | 584 |
| Crystal size [mm³] | 0.2 x 0.15 x 0.15 |
| θ range [°] | 2.50 → 27.3°. |
| Reflections collected | 9836 |
| Independent reflections | 6343 [R(int) = 0.0430] |
| S | 1.043 |
| R [I>2σ(I)] | R1 = 0.0796, wR2 = 0.1621 |
| R indices (all data) | R1 = 0.1761, wR2 = 0.2009 |
| Extinction coefficient | 0.044(4) |

| | |
|---|---|
| Fw = formula weight; T = Temperature of data collection; λ = wavelength of X-ray source; Dₘ = calculated density; θ range = Theta range of data collection; S = Goodness-o- fit on F²; R [I>2σ(I)] = Final R indices [I>2sigma(I)] | |

**Table 3.2 Single crystal data and structure refinement of HBr2 = bromide, Form II (trihydrate)**

| | |
|---|---|
| Empirical formula | C₃₁H₃₄N₅O₄⁺ • Br⁻ • 3 H₂O |
| Fw | 674.59 |
| T [K] | 293(2) |
| λ [Å] | 0.71073 |
| Crystal system | Triclinic |
| Space group | P-1 |
| Unit cell dimensions | |
| a [Å] | 9.2250(2) |
| b [Å] | 12.4280(2) |
| c [Å] | 15.4280(3) |
| α [°] | 103.8360(7) |
| β [°] | 101.4230(8) |
| γ [°] | 96.1460(7) |
| V [Å³] | 1661.14(5) |
| Z | 2 |
| Dₘ [g/cm³] | 1.349 |
| F(000) | 704 |
| Crystal size [mm³] | 0.2 x 0.2 x 0.2 |
| θ range [°] | 3.00 → 27.5 |
| Reflections collected | 12767 |
| Independent reflections | 7487 [Rᵢₙₜ = 0.0250] |
| S | 1.041 |
| R [I>2σ(I)] | R1 = 0.0452, wR2 = 0.0947 |
| R indices (all data) | R1 = 0.0703, wR2 = 0.1076 |
| Fw = formula weight; T = Temperature of data collection; λ = wavelength of X-ray source; Dₘ = calculated density; θ range = Theta range of data collection; S = Goodness-o- fit on F²; R [I>2σ(I)] = Final R indices [I>2sigma(I)] | |

**Table 3.3 Single crystal data and structure refinement of Pho2 = phosphate, Form II (3,5 - hydrate)**

| | |
|---|---|
| Empirical formula | C₃₁H₃₄N₅O₄⁺ • H₂PO₄⁻ • 3.5 H₂O |
| Fw | 700.67 |
| T [K] | 293(2) |
| λ [Å] | 0.71073 |
| Crystal system | Monoclinic |
| Space group | P 2₁/c |
| Unit cell dimensions | |
| a [Å] | 17.1960(3) |
| b [Å] | 11.8290(2) |
| c [Å] | 37.7530(6) |
| β [°] | 116.007(2) |
| V [Å³] | 6901.8(2) |
| Z | 8 |
| Dₘ [g/cm ³] | 1.349 |
| F(000) | 2968 |
| Crystal size [mm³] | 0.2 x 0.2 x 0.1 |
| θ range [°] | 1.3 → 22.5 |
| Reflections collected | 16435 |
| Independent reflections | 9001 [Rᵢₙₜ = 0.0518] |
| S | 1.081 |
| R [I>2σ(I)] | R1 = 0.0860, wR2 = 0.2095 |
| R indices (all data) | R1 = 0.1217, wR2 = 0.2314 |

| | |
|---|---|
| Fw = formula weight; T = Temperature of data collection; λ = wavelength of X-ray source; Dₘ = calculated density; θ range = Theta range of data collection; S = Goodness-o- fit on F²; R [I>2σ(I)] = Final R indices [I>2sigma(I)] | |

**Table 3.4 Single crystal data and structure refinement of Mes1 = mesylate, Form I (= hemihydrate)**

| | |
|---|---|
| Empirical formula | C₃₂H₃₄N₅O₄⁺ • CH₃SO₃⁻ • 0.5 H₂O |
| Fw | 644.73 |
| T [K] | 293(2) |
| λ[Å] | 0.71073 |
| Crystal system | Triclinic |
| Space group | P -1 |
| Unit cell dimensions | |
| a [Å] | 11.3700(6) |
| b [Å] | 16.4940(6) |
| c [Å] | 19.0370(8) |
| α [°] | 68.124(3) |
| β [°] | 84.892(2) |
| γ [°] | 89.884(2) |
| V [Å³] | 3298.0(3) |
| Z | 4 |
| Dₘ [g/cm³] | 1.298 |
| F(000) | 1364 |
| Crystal size [mm³] | 0.15 x 0.12 x 0.1 |
| θ range [°] | 2 → 24 |
| Reflections collected | 11140 |
| Independent reflections | 8267 [Rᵢₙₜ = 0.0541] |
| S | 1.080 |
| R [I>2σ(I)] | R1 = 0.0746, wR2 = 0.1635 |
| R indices (all data) | R1 = 0.1210, wR2 = 0.1889 |

| | |
|---|---|
| Fw = formula weight; T = Temperature of data collection; λ = wavelength of X-ray source; Dₘ = calculated density; θ range = Theta range of data collection; S = Goodness-o- fit on F²; R [I>2σ(I)] = Final R indices [I>2sigma(I)] | |

**Table 3.5 Single crystal data and structure refinement of Ise1 = isethionate, Form I (= anhydrous form)**

| | |
|---|---|
| Empirical formula | C₃₁H₃₄N₅O₄⁺ • C₂H₆SO₄⁻ |
| Fw | 665.75 |
| T [K] | 293(2) |
| λ[Å] | 0.71073 |
| Crystal system | Monoclinic |
| Space group | P 2₁/c |
| Unit cell dimensions | |
| a [Å] | 18.2760(4) |
| b [Å] | 10.3290(7) |
| c [Å] | 19.1750(8) |
| β [°] | 112.934(2) |
| V [Å³] | 3333.6(3) |
| Z | 4 |
| Dₘ [g/cm³] | 1.327 |
| Absorption coefficient | 0.155 |
| F(000) | 1408 |
| Crystal size [mm³] | 0.2 x 0.15 x 0.05 |
| θ range [°] | 2.25 → 24.25 |
| Reflections collected | 13587 |
| Independent reflections | 5148 [R(int) = 0.1346] |
| Goodness-of-fit on F² | 1.110 |
| R [I>2σ(I)] | R1 = 0.1014, wR2 = 0.1324 |
| R indices (all data) | R1 = 0.2081, wR2 = 0.1566 |

| | |
|---|---|
| Fw = formula weight; T = Temperature of data collection; λ = wavelength of X-ray source; Dₘ = calculated density; θ range = Theta range of data collection; S = Goodness-o- fit on F²; R [I>2σ(I)] = Final R indices [I>2sigma(I)] | |

**Table 3.6 Crystal data and structure refinement of lse5 = isethionate, Form V (= dihydrate)**

| | |
|---|---|
| Empirical formula | C₃₁H₃₄N₅O₄⁺ • C₂H₆SO₄⁻ |
| Fw | 665.75 |
| T [K] | 293(2) |
| λ[Å] | 0.71073 |
| Crystal system | Monoclinic |
| Space group | P 2₁/c |
| Unit cell dimensions | |
| a [Å] | 18.2760(4) |
| b [Å] | 10.3290(7) |
| c [Å] | 19.1750(8) |
| β [°] | 112.934(2) |
| V [Å³] | 3333.6(3) |
| Z | 4 |
| Dₘ [g/cm³] | 1.327 |
| Absorption coefficient | 0.155 |
| F(000) | 1408 |
| Crystal size [mm³] | 0.2 x 0.15 x 0.05 |
| θ range [°] | 2.25 → 24.25 |
| Reflections collected | 13587 |
| Independent reflections | 5148 [R(int) **=** 0.1346] |
| S | 1.110 |
| R [I>2σ(I)] | R1 = 0.1014, wR2 = 0.1324 |
| R indices (all data) | R1 = 0.2081, wR2 = 0.1566 |

| | |
|---|---|
| Fw = formula weight; T = Temperature of data collection; λ = wavelength of X-ray source; Dₘ = calculated density; θ range = Theta range of data collection; S = Goodness-o- fit on F²; R [I>2σ(I)] = Final R indices [I>2sigma(I)] | |

**Table 3.7 Crystal data and structure refinement of Bes1 = besylate, Form I (= trihydrate)**

| | |
|---|---|
| Empirical formula | C₃₁H₃₄N₅O₄⁺ • C₆H₅SO₃⁻ • 3 H₂O |
| Fw | 751.84 |
| T [K] | 293(2) |
| λ[Å] | 0.71073 |
| Crystal system | Triclinic |
| Space group | P -1 |
| Unit cell dimensions | |
| a [Å] | 9.2700(2) |
| b [Å] | 11.8820(3) |
| c [Å] | 17.3410(5) |
| α [°] | 86.4650(11) |
| β [°] | 89.0940(11) |
| γ [°] | 81.3340(17) |
| V [Å³] | 1884.60(8) |
| Z | 2 |
| Dₘ [g/cm³] | 1.325 |
| F(000) | 796 |
| Crystal size [mm³] | 0.6 x 0.2 x 0.1 |
| θ range[°] | 2 → 25 |
| Reflections collected | 11462 |
| Independent reflections | 6607 [Rᵢₙₜ = 0.0267] |
| S | 1.025 |
| R [I>2σ(I)] | R1 = 0.0565, wR2 = 0.1401 |
| R indices (all data) | R1 = 0.0794, wR2 = 0.1543 |

| | |
|---|---|
| Fw = formula weight; T = Temperature of data collection; λ = wavelength of X-ray source; Dₘ = calculated density; θ range = Theta range of data collection; S = Goodness-o- fit on F²; R [I>2σ(I)] = Final R indices [I>2sigma(I)] | |

**Table 3.8 Crystal data and structure refinement of Tos1 = tosylate, Form I (= monohydrate)**

| | |
|---|---|
| Empirical formula | C₃₈H₃₄N₅O₄⁺ • C₇H₇O₃S⁻ • H₂O |
| Fw | 729.83 |
| T [K] | 293(2) |
| λ[Å] | 0.71073 |
| Crystal system | Triclinic |
| Space group | P-1 |
| Unit cell dimensions | |
| a [Å] | 10.0670(8) |
| b [Å] | 11.5880(9) |
| c [Å] | 17.4860(19) |
| α [°] | 85.599(4) |
| β [°] | 88.670(5) |
| γ [°] | 68.083(9) |
| V [Å³] | 1886.8(3) |
| Z | 2 |
| Dₘ [g/cm³] | 1.285 |
| F(000) | 772 |
| Crystal size [mm³] | 0.15 x 0.1 x 0.01 |
| θ range[°] | 2 → 23. |
| Reflections collected | 4871 |
| Independent reflections | 3713 [Rᵢₙₜ = 0.0789] |
| S | 1.065 |
| R [I>2σ(I)] | R1 = 0.1187, wR2 = 0.2008 |
| R indices (all data) | R1 = 0.2506, wR2 = 0.2529 |
| Fw = formula weight; T = Temperature of data collection; λ = wavelength of X-ray source; Dₘ = calculated density; θ range = Theta range of data collection; S = Goodness-o- fit on F²; R [I>2σ(I)] = Final R indices [1>2sigma(l)] | |

**Table 3.9 Crystal data and structure refinement of Tos2 = tosylate, Form II (= trihydrate)**

| | |
|---|---|
| Empirical formula | C₃₁H₃₄N₅O₄⁺ • C₆H₅O₃S⁻ • 3H₂O |
| Fw | 765.87 |
| T [K] | 293(2) |
| λ[Å] | 0.71073 |
| Crystal system | Triclinic |
| Space group | P-1 |
| Unit cell dimensions | |
| a [Å] | 9.2760(2) |
| b [Å] | 12.4080(2) |
| c [Å] | 17.1470(4) |
| α [°] | 87.634(1) |
| β [°] | 89.104(1) |
| γ [°] | 78.453(2) |
| V [Å³] | 1931.92(7) A |
| Z | 2 |
| Dₘ [g/cm³] | 1.317 |
| F(000) | 812 |
| Crystal size [mm³] | 0.2x0.15x0.1 |
| θ range[°] | 2.2 → 27.5 |
| Reflections collected | 13198 |
| Independent reflections | 8761 [R(int) = 0.0238] |
| S | 1.019 |
| R [I>2σ(I)] | R1 = 0.0560, wR2 = 0.1301 |
| R indices (all data) | R1 = 0.0831, wR2 = 0.1448 |

| | |
|---|---|
| Fw = formula weight; T = Temperature of data collection; λ = wavelength of X-ray source; Dₘ = calculated density; θ range = Theta range of data collection; S = Goodness-o- fit on F²; R [I>2σ(I)] = Final R indices [I>2sigma(I)] | |

**Table 3.10 Crystal data and structure refinement of Cas3 = camphorsulfonate, Form III (= anhydrous form)**

| | |
|---|---|
| Empirical formula | C₃₁H₃₄N₅O₄⁺ • C₁₀H₁₅SO₄⁻ |
| Fw | 771.91 |
| T [K] | 293(2) |
| λ [Å] | 0.71073 |
| Crystal system | Triclinic |
| Space group | P-1 |
| Unit cell dimensions | |
| a [Å] | 9.7410(3) |
| b [Å] | 12.5540(4) |
| c [Å] | 17.6910(6) |
| α [°] | 73.3620(12) |
| β [°] | 75.9590(12) |
| γ [°] | 80.0360(17) |
| V [Å³] | 1998.38(11) |
| Z | 2 |
| Dₘ [g/cm³] | 1.283 |
| F(000) | 820 |
| Crystal size [mm³] | 0.25 x 0.2 x 0.15 |
| θ range [°] | 2.6 → 24.5. |
| Reflections collected | 11336 |
| Independent reflections | 6629 [Rᵢₙₜ = 0.0423] |
| S | 1.106 |
| R [I>2σ(I)] | R1 = 0.0701, wR2 = 0.1452 |
| R indices (all data) | R1 = 0.1073, wR2 = 0.1585 |
| Extinction coefficient | 0.0231 (13) |

| | |
|---|---|
| Fw = formula weight; T = Temperature of data collection; λ = wavelength of X-ray source; Dₘ = calculated density; θ range = Theta range of data collection; S = Goodness-o- fit on F²; R [I>2σ(I)] = Final R indices [I>2sigma(I)] | |

**Table 3.11 Crystal data and structure refinement of Cas5 = camphorsulfonate, Form V (= hemisolvate with propionitrile)**

| | |
|---|---|
| Empirical formula | C₃₁H₃₄N₅O₄ • C₁₀H₁₅O₄S⁻ • 0.5 C₃H₅N |
| Fw | 799.45 |
| T [K] | 293(2) |
| λ [Å] | 0.71073 |
| Crystal system | Triclinic |
| Space group | P 1 |
| Unit cell dimensions | |
| a [Å] | 9.5050(5) |
| b [Å] | 13.1300(7) |
| c [Å] | 17.903(2) |
| α [°] | 78.389(2) |
| β [°] | 76.262(2) |
| γ [°] | 89.425(4) |
| V [Å³] | 2124.3(2) |
| Z | 2 |
| Dₘ [g/cm³] | 1.250 |
| F(000) | 850 |
| Crystal size [mm³] | 0.4 x 0.2 x 0.2 |
| θ range [°] | 2.2 → 25.5 |
| Reflections collected | 11323 |
| Independent reflections | 11323 [Rᵢₙₜ = 0.0000] |
| S | 1.067 |
| R [I>2σ(I)] | R1 = 0.0603, wR2 = 0.1672 |
| R indices (all data) | R1 = 0.0681, wR2 = 0.1772 |
| Absolute structure parameter | 0.03(9) |

| | |
|---|---|
| Fw = formula weight; T = Temperature of data collection; λ = wavelength of X-ray source; Dₘ = calculated density; θ range = Theta range of data collection; S = Goodness-o- fit on F²; R [I>2σ(I)] = Final R indices [I>2sigma(I)] | |

**Table 3.12 Crystal data and structure refinement of Cas12 = camphorsulfonate, Form XII (= hemisolvate with 1,2-dimethoxyethane)**

| | |
|---|---|
| Empirical formula | C₃₁H₃₄N₅O₄⁺ • C₁₀H₁₅O₄S⁻ • 0.5 C₄H₁₀O₂ |
| Fw | 816.97 |
| T [K] | 293(2) |
| λ[Å] | 0.71073 |
| Crystal system | Triclinic |
| Space group | P 1 |
| Unit cell dimensions | |
| a [Å] | 10.854(1) |
| b [Å] | 11.289(1) |
| c [Å] | 18.421 (2) |
| α [°] | 105.02(1) |
| β[°] | 96.38(1) |
| γ [°] | 104.27(1) |
| V [Å³] | 2075.5(2) |
| Z | 2 |
| Dₘ[g/cm³] | 1.307 |
| F(000) | 870 |
| Crystal size [mm³] | 0.3 x 0.2 x 0.15 |
| θ range [°] | 3 → 26 |
| Reflections collected | 7261 |
| Independent reflections | 7261 [R(int) = 0.0000] |
| S | 1.058 |
| R [I>2σ(I)] | R1 = 0.0680, wR2 = 0.1378 |
| R indices (all data) | R1 = 0.1069, wR2 = 0.1550 |
| Absolute structure parameter | -0.12(13) |
| Fw = formula weight; T = Temperature of data collection; λ = wavelength of X-ray source; Dₘ = calculated density; θ range = Theta range of data collection; S = Goodness-o- fit on F²; R [I>2σ(I)] = Final R indices [I>2sigma(I)] | |

**Table 3.13 Crystal data and structure refinement of Cit2 = citrate, Form II (= dihydrate)**

| | |
|---|---|
| Empirical formula | 2 (C₃₁H₃₄N₅O₄⁺) • C₆H₆O₇²⁻ • 2 H₂O |
| Fw | 1307.4 |
| T [K] | 293(2) K |
| λ [Å] | 0.71073 Å |
| Crystal system | Triclinic |
| Space group | P-1 |
| Unit cell dimensions | |
| a [Å] | 12.2450(3) |
| b [Å] | 15.4250(4) |
| c [Å] | 18.6640(6) |
| α [°] | 77.523(1) |
| β [°] | 78.303(1) |
| γ [°] | 88.705(2) |
| V [Å³] | 3369.68(16) |
| Z | 2 |
| Dₘ [g/cm³] | 1.289 |
| F(000) | 1384 |
| Crystal size [mm³] | 0.25 x 0.18 x 01 |
| θ range [°] | 2 → 22.5 |
| Reflections collected | 14407 |
| Independent reflections | 8753 [Rᵢₙₜ = 0.0320] |
| S | 1.047 |
| R [I>2σ(I)] | R1 = 0.0859, wR2 = 0.2323 |
| R indices (all data) | R1 = 0.1163, wR2 = 0.2611 |
| Extinction coefficient | 0.020(3) |

| | |
|---|---|
| Fw = formula weight; T = Temperature of data collection; λ = wavelength of X-ray source; Dₘ = calculated density; θ range = Theta range of data collection; S = Goodness-o- fit on F²; R [I>2σ(I)] = Final R indices [I>2sigma(I)] | |

**Table 3.14 Crystal data and structure refinement of L-Lac1 = L-lactate, Form I (= 2.5 - hydrate)**

| | |
|---|---|
| Empirical formula | C₃₁H₃₄N₅O₄⁺ • C₃H₄O₃⁻ • 2.5 H₂O |
| Fw | 674.74 |
| T [K] | 293(2) |
| λ[Å] | 0.71073 |
| Crystal system | Monoclinic |
| Space group | P 2₁ |
| Unit cell dimensions | |
| a [Å] | 16.8380(6) |
| b [Å] | 11.7710(9) |
| c [Å] | 17.3490(12) |
| β [°] | 91.819(3) |
| V [Å³] | 3436.8(4) |
| Z | 4 |
| Dₘ [g/cm³] | 1.304 |
| F(000) | 1436 |
| Crystal size [mm³] | 0.15 x 0.1 x 0.05 |
| θ range [°] | 2.5 → 26.00 |
| Reflections collected | 14324 |
| Independent reflections | 10572 [Rᵢₙₜ = 0.0709] |
| S | 1.042 |
| R [I>2σ(I)] | R1 = 0.0881, wR2 = 0.1583 |
| R indices (all data) | R1 = 0.1950, wR2 = 0.1977 |
| Absolute structure parameter | 0.8(19) |
| Extinction coefficient | 0.0110(11) |

| | |
|---|---|
| Fw = formula weight; T = Temperature of data collection; λ = wavelength of X-ray source; Dₘ = calculated density; θ range = Theta range of data collection; S = Goodness-o- fit on F²; R [I>2σ(I)] = Final R indices [I>2sigma(I)] | |

**Table 3.15 Crystal data and structure refinement of L-Mal3 = L-malate, Form lll (= tetrahydrate)**

| | |
|---|---|
| Empirical formula | 2 (C₃₁H₃₄N₅O₄⁺)• C₄H₄O₅²⁻ • 4 H₂O |
| Fw | 1285.40 |
| T [K] | 293(2) |
| λ [Å] | 0.71073 |
| Crystal system | Triclinic |
| Space group | P1 |
| Unit cell dimensions | |
| a [Å] | 9.2360(3) |
| b [Å] | 11.1340(4) |
| c [Å] | 16.5470(6) |
| α [°] | 86.3520(19) |
| β [°] | 74.7070(19) |
| γ [°] | 84.372(2) |
| V [Å³] | 1632.16(10) |
| Z | 1 |
| Dₘ [g/cm³] | 1.308 |
| F(000) | 682 |
| Crystal size [mm³] | 0.15 x 0.1 x 0.06 |
| θ range [°] | 3 → 22.50 |
| Reflections collected | 7968 |
| Independent reflections | 7968 [Rᵢₙₜ = 0.0000] |
| S | 1.064 |
| R [I>2σ(I)] | R1 = 0.0711, wR2 = 0.1242 |
| R indices (all data) | R1 = 0.1215, wR2 = 0.1458 |

| | |
|---|---|
| Fw = formula weight; T = Temperature of data collection; λ = wavelength of X-ray source; Dₘ = calculated density; θ range = Theta range of data collection; S = Goodness-o- fit on F²; R [I>2σ(I)] = Final R indices [I>2sigma(I)] | |

**Table 3.16 Crystal data and structure refinement of Suc3 = succinate, Form lll (= hexahydrate)**

| | |
|---|---|
| Empirical formula | 2(C₃₁H₃₄N₅O₄⁺) • C₄H₄O₄²⁻ • 6H₂O |
| Fw | 1305.44 |
| T [K] | 293(2) |
| λ[Å] | 0.71073 |
| Crystal system | Triclinic |
| Space group | P-1 |
| Unit cell dimensions | |
| a [Å] | 8.9690(4) |
| b [Å] | 12.0910(6) |
| c [Å] | 17.037(2) |
| α [°] | 95.004(3) |
| β [°] | 103.678(3) |
| γ [°] | 109.813(5) |
| V [Å³] | 1660.35(15) |
| Z | 1 |
| Dₘ [g/cm³] | 1.306 |
| F(000) | 694 |
| Crystal size [mm³] | 0.3 x 0.15 x 0.05 |
| θ range [°] | 3.5 → 27.4 |
| Reflections collected | 6180 |
| Independent reflections | 4915 [R(ᵢₙₜ) = 0.0418] |
| S | 1.024 |
| R [I>2σ(I)] | R1 = 0.0747, wR2 = 0.1213 |
| R indices (all data) | R1 = 0.1662, wR2 = 0.1478 |
| Extinction coefficient | 0.035(3) |

| | |
|---|---|
| Fw = formula weight; T = Temperature of data collection; λ = wavelength of X-ray source; Dₘ = calculated density; θ range = Theta range of data collection; S = Goodness-o- fit on F²; R [I>2σ(I)] = Final R indices [I>2sigma(I)] | |

**Table 3.17 Crystal data and structure refinement of Gen11 = gentisate, Form XI (= hemihydrate)**

| | |
|---|---|
| Empirical formula | C₃₁H₃₄N₅O₄ • C₇H₅O₄ 0.5 • H₂O |
| Fw | 702.75 |
| T [K] | 293(2) |
| λ [Å] | 0.71073 |
| Crystal system | Triclinic |
| Space group | P-1 |
| Unit cell dimensions | |
| a [Å] | 14.919(2) |
| b [Å] | 15.713(2) |
| c [Å] | 17.136(2) |
| α [°] | 93.770(3) |
| β [°] | 113.268(4) |
| γ [°] | 104.383(3) |
| V [Å³] | 3513.5(5) |
| Z | 4 |
| Dₘ [g/cm³] | 1.329 |
| F(000) | 1484 |
| Crystal size [mm³] | 0.25 x 0.25 x 0.2 |
| θ range [°] | 2 25 |
| Reflections collected | 19141 |
| Independent reflections | 11651 [Rᵢₙₜ = 0.0451] |
| S | 1.203 |
| R [I>2σ(I)] | R1 = 0.1079, wR2 = 0.1734 |
| R indices (all data) | R1 = 0.1712, wR2 = 0.1958 |

| | |
|---|---|
| Fw = formula weight; T = Temperature of data collection; λ = wavelength of X-ray source; Dₘ = calculated density; θ range = Theta range of data collection; S = Goodness-o- fit on F²; R [I>2σ(I)] = Final R indices [I>2sigma(I)] | |

**Table 3.18 Crystal data and structure refinement of Man1 = mandelate, Form I (= monohydrate)**

| | |
|---|---|
| Empirical formula | C₃₁H₃₄N₅O₄⁺ • C₈H₇O₃⁻ • H₂O |
| Fw | 709.78 |
| T [K] | 293(2) |
| λ [Å] | 0.71073 |
| Crystal system | Triclinic |
| Space group | P 1 |
| Unit cell dimensions | |
| a [Å] | 9.9160(3) |
| b [Å] | 11.2690(4) |
| c [Å] | 16.6690(7) |
| α [°] | 89.0210(13) |
| β [°] | 82.4760(13) |
| γ [°] | 76.969(2) |
| V [Å³] | 1798.91(11) |
| Z | 2 |
| Dₘ [g/cm³] | 1.310 |
| F(000) | 752 |
| Crystal size [mm³] | 0.25 x 0.2 x 0.1 |
| θ range [°] | 2.3 → 27.4 |
| Reflections collected | 12776 |
| Independent reflections | 12776 [R(ᵢₙₜ) = 0.0000] |
| S | 1.036 |
| R [I>2σ(I)] | R1 = 0.0806, wR2 = 0.1606 |
| R indices (all data) | R1 = 0.1481, wR2 = 0.1928 |
| Absolute structure parameter | -0.5(16) |

| | |
|---|---|
| Fw = formula weight; T = Temperature of data collection; λ = wavelength of X-ray source; Dₘ = calculated density; θ range = Theta range of data collection; S = Goodness-o- fit on F²; R [I>2σ(I)] = Final R indices [1>2sigma(l)] | |

**Table 3.19 Crystal data and structure refinement of Sal2 = salicylate, Form II (= anhydrous form)**

| | |
|---|---|
| Empirical formula | C₃₁H₃₄N₅O₄⁺ • C₇H₅O₃⁻ |
| Fw | 677.74 |
| T [K] | 293(2) |
| λ [Å] | 0.71073 |
| Crystal system | Triclinic |
| Space group | P-1 |
| Unit cell dimensions | |
| a [Å] | 9.8770(2) |
| b [Å] | 13.4820(3) |
| c [Å] | 13.6460(3) |
| α [°] | 93.8310(10) |
| β[°] | 99.2880(10) |
| γ [°] | 103.131 (2) |
| V [Å³] | 1736.3(1) |
| Z | 2 |
| Dₘ [g/cm³] | 1.296 |
| F(000) | 716 |
| Crystal size [mm³] | 0.3 x 0.2 x 0.1 |
| θ range [°] | 2 → 25 |
| Reflections collected | 10009 |
| Independent reflections | 6037 [Rᵢₙₜ = 0.0233] |
| S | 1.040 |
| R [I>2σ(I)] | R1 = 0.0543, wR2 = 0.1304 |
| R indices (all data) | R1 = 0.0713, wR2 = 0.1438 |

| | |
|---|---|
| Fw = formula weight; T = Temperature of data collection; λ = wavelength of X-ray source; Dₘ = calculated density; θ range = Theta range of data collection; S = Goodness-o- fit on F²; R [I>2σ(I)] = Final R indices [I>2sigma(I)] | |

**Table 3.20 Crystal data and structure refinement of Xin1 = xinafoate, Form I (= monohydrate)**

| | |
|---|---|
| Empirical formula | C₃₁H₃₄N₅O₄⁺ • C₁₁H₉O₃⁻ • H₂O |
| Fw | 745.81 |
| T [K] | 293(2) |
| λ [Å] | 0.71073 |
| Crystal system | Monoclinic |
| Space group | P 2₁/C |
| Unit cell dimensions | |
| a [Å] | 11.0350(2) |
| b [Å] | 31.2480(7) |
| c [Å] | 11.3790(3) |
| β [°] | 94.298(1) |
| V [Å³] | 3912.69(15) |
| Z | 4 |
| Dₘ [g/cm³] | 1*.*266 |
| F(000) | 1576 |
| Crystal size [mm³] | 0.4 x 0.2 x 0.1 |
| θ range [°] | 2 → 24.5 |
| Reflections collected | 19328 |
| Independent reflections | 6429 [Rᵢₙₜ = 0.0497] |
| S | 1.033 |
| R [I>2σ(I)] | R1 = 0.0622, wR2 = 0.1307 |
| R indices (all data) | R1 = 0.1066, wR2 = 0.1498 |

| | |
|---|---|
| Fw = formula weight; T = Temperature of data collection; λ = wavelength of X-ray source; Dₘ = calculated density; θ range = Theta range of data collection; S = Goodness-o- fit on F²; R [I>2σ(I)] = Final R indices [I>2sigma(I)] | |

Thus, in accordance with one preferred embodiment, the present invention relates to the following salts and/or crystalline forms and/or crystalline salt forms of the compound 3-Z-[1-(4-(N-((4-methyl-piperazin-1-yl)-methylcarbonyl)-N-methyl-amino)-anilino)-1-phenyl-methylene]-6-methoxycarbonyl-2-indolinone:
Crystalline 3-Z-[1-(4-(N-((4-methyl-piperazin-1-yl)-methylcarbonyl)-N-methyl-amino)-anilino)-1-phenyl-methylene]-6-methoxycarbonyl-2-indolinone, in particular in hemihydrated form, characterized by a triclinic elementary cell with single crystal X-ray characteristic values of a, b, c, α, β, γ and of the cell volume V as given in above Table 3.1;
3-Z-[1-(4-(N-((4-methyl-piperazin-1-yl)-methylcarbonyl)-N-methyl-amino)-anilino)-1-phenyl-methylene]-6-methoxycarbonyl-2-indolinone chloride, in particular in crystalline anhydrous Form I as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone chloride, in particular in crystalline hydrated Form II as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone bromide, in particular in crystalline hydrated Form II, more particularly in the form of its trihydrate, characterized by a triclinic elementary cell with single crystal X-ray characteristic values of a, b, c, α, β, γ and of the cell volume V as given in above Table 3.2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone bromide, in particular in crystalline anhydrous Form VII as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone bromide, in particular in crystalline anhydrous Form VIII as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone phosphate, in particular in crystalline Form I as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone phosphate, in particular in crystalline hydrated Form II, more particularly in its 3,5 - hydrate form, characterized by a monoclinic elementary cell with single crystal X-ray characteristic values of a, b, c, β and of the cell volume V as given in above Table 3.3;
3-Z-[1-(4-(N-((4-methyl-piperazin-1-yl)-methylcarbonyl)-N-methyl-amino)-anilino)-1-phenyl-methylene]-6-methoxycarbonyl-2-indolinone sulfate, in particular in its crystalline hydrated Form I as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone sulfate, in particular in its crystalline Form V as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone sulfate, in particular in its crystalline hydrated Form VI as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone sulfate, in particular in its crystalline hydrated Form VII as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone methanesulfonate, in particular in crystalline hydrated Form I, more particularly in its hemihydrate form, characterized by a triclinic elementary cell with single crystal X-ray characteristic values of a, b, c, α, β, γ and of the cell volume V as given in above Table 3.4;
3-Z-[1-(4-(N-((4-methyl-piperazin-1-yl)-methylcarbonyl)-N-methyl-amino)-anilino)-1-phenyl-methylene]-6-methoxycarbonyl-2-indolinone ethanedisulfonate, in particular in its crystalline Form I as characterized in above Table 2;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}ethanedisulfonate, in particular in its crystalline Form I as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone ethanedisulfonate, in particular in its crystalline Form II as characterized in above Table 2;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}ethanedisulfonate, in particular in its crystalline Form II as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone ethanedisulfonate, in particular in its crystalline hydrated Form III as characterized in above Table 2;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}ethanedisulfonate, in particular in its crystalline hydrated Form III as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone ethanedisulfonate, in particular in its crystalline Form V as characterized in above Table 2;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}ethanedisulfonate, in particular in its crystalline Form V as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone ethanedisulfonate, in particular in its crystalline Form VI as characterized in above Table 2;
Bis{3-Z-[1-(4-(N-((4-methyl-piperazin-1-yl)-methylcarbonyl)-N-methyl-amino)-anilino)-1-phenyl- methylene] -6- methoxycarbonyl- 2- indolinone}ethanedisulfonate, in particular in its crystalline Form VI as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone ethanedisulfonate, in particular in its crystalline anhydrous Form VII as characterized in above Table 2;
Bis {3-Z-[1-(4-(N-((4-methyl-piperazin-1-yl)-methylcarbonyl)-N-methyl-amino)-anilino)-1-phenyl-methylene]-6-methoxycarbonyl-2-indolinone}ethanedisulfonate, in particular in its crystalline anhydrous Form VII as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone isethionate, in particular in crystalline anhydrous Form I, characterized by a monoclinic elementary cell with single crystal X-ray characteristic values of a, b, c, β and of the cell volume V as given in above Table 3.5;
3-Z-[1-(4-(N-((4-methyl-piperazin-1-yl)-methylcarbonyl)-N-methyl-amino)-anilino)-1-phenyl-methylene]-6-methoxycarbonyl-2-indolinone isethionate, in particular in its crystalline Form II as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone isethionate, in particular in its crystalline Form IV as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone isethionate, in particular in crystalline hydrated Form V, more particularly in its dihydrate form, characterized by a monoclinic elementary cell with single crystal X-ray characteristic values of a, b, c, β and of the cell volume V as given in above Table 3.5;
3-Z-[1-(4-(N-((4-methyl-piperazin-1-yl)-methylcarbonyl)-N-methyl-amino)-anilino)-1-phenyl-methylene]-6-methoxycarbonyl-2-indolinone benzenesulfonate, in particular in crystalline hydrated Form I, more particularly in its trihydrate form, characterized by a triclinic elementary cell with single crystal X-ray characteristic values of a, b, c, α, β, γ and of the cell volume V as given in above Table 3.7;
3-Z-[1-(4-(N-((4-methyl-piperazin-1-yl)-methylcarbonyl)-N-methyl-amino)-anilino)-1-phenyl-methylene]-6-methoxycarbonyl-2-indolinone benzenesulfonate, in particular in its crystalline Form III as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone benzenesulfonate, in particular in its crystalline Form V as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone tosylate, in particular in crystalline hydrated Form I, more particularly in its monohydrate form, characterized by a triclinic elementary cell with single crystal X-ray characteristic values of a, b, c, α, β, γ and of the cell volume V as given in above Table 3.8;
3-Z-[1-(4-(N-((4-methyl-piperazin-1-yl)-methylcarbonyl)-N-methyl-amino)-anilino)-1-phenyl-methylene]-6-methoxycarbonyl-2-indolinone tosylate, in particular in crystalline hydrated Form II, more particularly in its trihydrate form, characterized by a triclinic elementary cell with single crystal X-ray characteristic values of a, b, c, α, β, γ and of the cell volume V as given in above Table 3.9;
3-Z-[1-(4-(N-((4-methyl-piperazin-1-yl)-methylcarbonyl)-N-methyl-amino)-anilino)-1-phenyl-methylene]-6-methoxycarbonyl-2-indolinone camphorsulfonate, in particular in its crystalline anhydrous Form II as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone camphorsulfonate, in particular in crystalline anhydrous Form III, characterized by a triclinic elementary cell with single crystal X-ray characteristic values of a, b, c, α, β, γ and of the cell volume V as given in above Table 3.10;
3-Z-[1-(4-(N-((4-methyl-piperazin-1-yl)-methylcarbonyl)-N-methyl-amino)-anilino)-1-phenyl-methylene]-6-methoxycarbonyl-2-indolinone camphorsulfonate, in particular in crystalline solvated Form V, more particularly in its hemisolvated form with propionitrile, characterized by a triclinic elementary cell with single crystal X-ray characteristic values of a, b, c, α, β, γ and of the cell volume V as given in above Table 3.11;
3-Z-[1-(4-(N-((4-methyl-piperazin-1-yl)-methylcarbonyl)-N-methyl-amino)-anilino)-1-phenyl-methylene]-6-methoxycarbonyl-2-indolinone camphorsulfonate, in particular in crystalline solvated Form XII, more particularly in its hemisolvated form with 1,2-dimethoxyethane, characterized by a triclinic elementary cell with single crystal X-ray characteristic values of a, b, c, α, β, γ and of the cell volume V as given in above Table 3.12;
3-Z-[1-(4-(N-((4-methyl-piperazin-1-yl)-methylcarbonyl)-N-methyl-amino)-anilino)-1-phenyl-methylene]-6-methoxycarbonyl-2-indolinone naphthalene-1,5-disulfonate, in particular in its crystalline Form III as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone naphthalene-1,5-disulfonate, in particular in its crystalline Form IV as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone naphthalene-1,5-disulfonate, in particular in its crystalline hydrated Form V as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone citrate, in particular in its crystalline anhydrous Form I as characterized in above Table 2;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}citrate, in particular in its crystalline hydrated Form II, more particularly in its dihydrated form, characterized by a triclinic elementary cell with single crystal X-ray characteristic values of a, b, c, α, β, γ and of the cell volume V as given in above Table 3.13;
3-Z-[1-(4-(N-((4-methyl-piperazin-1-yl)-methylcarbonyl)-N-methyl-amino)-anilino)-1-phenyl-methylene]-6-methoxycarbonyl-2-indolinone D-tartrate, in particular in its crystalline hydrated Form I as characterized in above Table 2;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}D-tartrate, in particular in its crystalline Form II as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone L-tartrate, in particular in its crystalline hydrated Form I as characterized in above Table 2;
Bis{3-Z-[1-(4-(N-((4-methyl-piperazin-1-yl)-methylcarbonyl)-N-methyl-amino)-anilino)-l-phenyl-methylene]-6-methoxycarbonyl-2-indolinone}L-tartrate, in particular in its crystalline Form II as characterized in above Table 2;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}fumarate, in particular in its crystalline hydrated Form I as characterized in above Table 2;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}fumarate, in particular in its crystalline anhydrous Form III as characterized in above Table 2;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}maleate, in particular in its crystalline anhydrous Form I as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone L-lactate, in particular in crystalline hydrated Form I, more particularly in its 2.5-hydrated form, characterized by a monoclinic elementary cell with single crystal X-ray characteristic values of a, b, c, β and of the cell volume V as given in above Table 3.14;
3-Z-[1-(4-(N-((4-methyl-piperazin-1-yl)-methylcarbonyl)-N-methyl-amino)-anilino)-1-phenyl-methylene]-6-methoxycarbonyl-2-indolinone glycolate, in particular in its crystalline hydrated Form I as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone glycinate, in particular in its crystalline Form I as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone L-malate, in particular in its crystalline hydrated Form I as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone L-malate, in particular in its crystalline Form II as characterized in above Table 2;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone} L-malate, in particular in its crystalline Form II as characterized in above Table 2;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}L-malate, in particular in its crystalline hydrated Form III, more particularly in its tetrahydrated form, characterized by a triclinic elementary cell with single crystal X-ray characteristic values of a, b, c, α, β, γ and of the cell volume V as given in above Table 3.15;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone. L-malate, in particular in its crystalline Form IV as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone D-malate, in particular in its crystalline hydrated Form I as characterized in above Table 2;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}D-malate, in particular in its crystalline Form IV as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone malonate, in particular in its crystalline hydrated Form I as characterized in above Table 2;
Bis{3-Z-[1-(4-(N-((4-methyl-piperazin-1-yl)-methylcarbonyl)-N-methyl-amino)-anilino)-l-phenyl-methylene]-6-methoxycarbonyl-2-indolinone}malonate, in particular in its crystalline hydrated Form I as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone malonate, in particular in its crystalline hydrated Form II as characterized in above Table 2;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}malonate, in particular in its crystalline hydrated Form II as characterized in above Table 2;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}malonate, in particular in its crystalline hydrated and/or solvated Form III as characterized in above Table 2;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone]malonate, in particular in its crystalline Form IV as characterized in above Table 2;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}malonate, in particular in its crystalline Form V as characterized in above Table 2;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}malonate, in particular in its crystalline Form VI as characterized in above Table 2;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}succinate, in particular in its crystalline hydrated Form I as characterized in above Table 2;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}succinate, in particular in its crystalline hydrated Form II as characterized in above Table 2;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}succinate, in particular in its crystalline hydrated Form III, more particularly in its hexahydrated form, characterized by a triclinic elementary cell with single crystal X-ray characteristic values of a, b, c, α, β, γ and of the cell volume V as given in above Table 3.16;
3-Z-[1-(4-(N-((4-methyl-piperazin-1-yl)-methylcarbonyl)-N-methyl-amino)-anilino)-1-phenyl-methylene]-6-methoxycarbonyl-2-indolinone oxalate, in particular in its crystalline hydrated Form III as characterized in above Table 2;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}oxalate, in particular in its crystalline hydrated Form III as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone oxalate, in particular in its crystalline Form V as characterized in above Table 2;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}oxalate, in particular in its crystalline Form V as characterized in above Table 2;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}oxalate, in particular in its crystalline hydrated Form VI as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone gentisate, in particular in its crystalline hydrated Form I as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone gentisate, in particular in crystalline hydrated Form XI, more particularly in its hemihydrated form, characterized by a triclinic elementary cell with single crystal X-ray characteristic values of a, b, c, α, β, γ and of the cell volume V as given in above Table 3.17;
3-Z-[1-(4-(N-((4-methyl-piperazin-1-yl)-methylcarbonyl)-N-methyl-amino)-anilino)-1-phenyl-methylene]-6-methoxycarbonyl-2-indolinone camphorate, in particular in its crystalline hydrated Form II as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone camphorate, in particular in its crystalline Form III as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone benzoate, in particular in its crystalline hydrated Form II as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone benzoate, in particular in its crystalline Form III as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone mandelate, in particular in crystalline hydrated Form I, more particularly in its monohydrated form, characterized by a triclinic elementary cell with single crystal X-ray characteristic values of a, b, c, α, β, γ and of the cell volume V as given in above Table 3.18;
3-Z-[1-(4-(N-((4-methyl-piperazin-1-yl)-methylcarbonyl)-N-methyl-amino)-anilino)-1-phenyl-methylene]-6-methoxycarbonyl-2-indolinone saccharinate, in particular in its crystalline hydrated Form II as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone saccharinate, in particular in its crystalline Form III as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone saccharinate, in particular in its crystalline hydrated Form V as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone salicylate, in particular in its crystalline Form I as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone salicylate, in particular in crystalline anhydrous Form II, characterized by a triclinic elementary cell with single crystal X-ray characteristic values of a, b, c, α, β, γ and of the cell volume V as given in above Table 3.19;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}L-aspartate, in particular in its crystalline hydrated Form I as characterized in above Table 2;
Bis{3-Z-[1-(4-(N-((4-methyl-piperazin-1-yl)-methylcarbonyl)-N-methyl-amino)-anilino)-l-phenyl-methylene]-6-methoxycarbonyl-2-indolinone}L-aspartate, in particular in its crystalline Form II as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone xinafoate, in particular in crystalline hydrated Form I, more particularly in its monohydrate form, characterized by a monoclinic elementary cell with single crystal X-ray characteristic values of a, b, c, β and of the cell volume V as given in above Table 3.20;
3-Z-[1-(4-(N-((4-methyl-piperazin-1-yl)-methylcarbonyl)-N-methyl-amino)-anilino)-1-phenyl-methylene]-6-methoxycarbonyl-2-indolinone ascorbate, in particular in its crystalline Form I as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone ascorbate, in particular in its crystalline Form III as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone ascorbate, in particular in its crystalline Form IV as characterized in above Table 2;
3-Z-[1-(4-(N-((4-methyl-piperazin-1-yl)-methylcarbonyl)-N-methyl-amino)-anilino)-1-phenyl-methylene]-6-methoxycarbonyl-2-indolinone ascorbate, in particular in its crystalline Form VI as characterized in above Table 2; and
3-Z-[1-(4-(N-((4-methyl-piperazin-1-yl)-methylcarbonyl)-N-methyl-amino)-anilino)-1-phenyl-methylene]-6-methoxycarbonyl-2-indolinone ascorbate, in particular in its crystalline Form VII as characterized in above Table 2.

A further object of the present invention is the use of the above salts and crystalline salt forms as medicament.

A further object of the present invention is the use of the above salts and crystalline salt forms as medicament for the treatment or prevention of the following diseases.

The diseases which can be treated by the salts and crystal salt forms of the compound of formula (I) in accordance with the present invention are all kind of diseases in which cell proliferation, migration or apoptosis of myeloma cells, or angiogenesis are involved, which can be of oncological nature such as all types of malignant neoplasias or cancers, or of non-oncological nature, such as diabetic retinopathy, rheumatoid arthritis, or psoriasis.

Among cancers, selected specific target indications are solid tumours, such as urogenital cancers (such as prostate cancer, renal cell cancers, bladder cancers), gynecological cancers (such as ovarian cancers, cervical cancers, endometrial cancers), lung cancer, gastrointestinal cancers (such as colorectal cancers, pancreatic cancer, gastric cancer, oesophageal cancers, hepatocellular cancers, cholangiocellular cancers), head and neck cancer, malignant mesothelioma, breast cancer, malignant melanoma or bone and soft tissue sarcomas, and haematologic neoplasias, such as multiple myeloma, acute myelogenous leukemia, chronic myelogenous leukemia, myelodysplastic syndrome and acute lymphoblastic leukemia. Of special interest is the treatment of hormone sensitive or hormone refractory prostate cancer, ovarian carcinoma, non small cell lung cancer, small cell lung cancer, or multiple myeloma. Preferably, the underlying mechanism by which the above-mentioned diseases may be treated is via antagonism of at least one receptor selected from VEGFR 1 to 3, PDGFRα and β, FGFR1, 2 and 3, EGFR, HER2, IGF1R, HGFR, c-Kit, or a src tyrosine kinase family member.

In a further embodiment, the diseases which can be treated by the salts and crystal salt forms of the compound of formula (I) in accordance with the present invention are also diseases which result from aberrant activity of the following tyrosine kinases: ABL, FGFR3, FLT3 and RET. Such diseases are, for example, Kidney Wilm's tumor, soft tissue osteosarcoma, glioblastoma, multiforme, Ph+ leukemias such as chronic myelogeneous leukemia (CML) or acute lymphocytic leukemia (ALL), epithelial cancers such as bladder and cervix cancers, multiple myeloma, hepatocellular carcinoma, skeletal abnormalities such as achondroplasia and hypochondraplasia, leukemias including acute myeloid leukemia (AML), AML with trilineage myelodysplasia (AML/TMDS), acute lymphoblastic leukemia (ALL), myelodysplastic syndrome (MDS), cancers of the nerve tissue such as neuroblastoma, multiple endocrine neoplasias type 2A and 2B (MEN2A and MEN2B), familial medullary thyroid carcinomas (FMTC), papillary thyroid carcinomas (PTC) and breast cancer.

In yet another embodiment, the diseases which may be treated with the salts or crystal salt forms of the compound of formula (I) in accordance with the present invention are immunologic disease or pathological condition involving an immunologic component. Such diseases are, for example, autoimmune diseases, for instance inflammatory diseases having an autoimmune component such as inflammatory diseases selected from inflammatory bowel disease (e.g. colitis ulcerosa and Morbus Crohn), rheumatoid arthritis, glomerulonephritis and lung fibrosis, psoriasis, psoriasis arthritis, hypersensitivity reactions of the skin, atherosclerosis, restenosis, asthma, multiple sclerosis and type 1 diabetes, and indications which need immunosuppressant therapy, for instance prevention or therapy of tissue or organ transplant rejection. Preferably, the underlying mechanism by which the above-mentioned diseases may be treated is via antagonism of the Lck tyrosine kinase, a tyrosine kinase belonging to the src family.

It has further been surprisingly found that the salts or crystal salt forms of the compound of formula (I) in accordance with the present invention are suitable for the treatment of specific fibrotic diseases selected from the group consisting of fibrosis and remodeling of lung tissue in chronic obstructive pulmonary disease, fibrosis and remodeling of lung tissue in chronic bronchitis, fibrosis and remodeling of lung tissue in emphysema, lung fibrosis and pulmonary diseases with a fibrotic component, fibrosis and remodeling in asthma, fibrosis in rheumatoid arthritis, virally induced hepatic cirrhosis, radiation-induced fibrosis, post angioplasty restenosis, chronic glomerulonephritis, renal fibrosis in patients receiving cyclosporine and renal fibrosis due to high blood pressure, diseases of the skin with a fibrotic component, and excessive scarring. Amongst these diseases, preferred diseases which my be treated with the salts or crystal salt forms of the compound of formula (I) in accordance with the present invention are lung fibrosis and pulmonary diseases with a fibrotic component selected from idiopathic pulmonary fibrosis, giant cell interstitial pneumonia, sarcodosis, cystic fibrosis, respiratory distress syndrome, drug-induced lung fibrosis, granulomatosis, silicosis, asbestosis, systemic scleroderma, the virally induced hepatic cirrhosis selected from hepatitis C induced hepatic cirrhosis, and the diseases of the skin with a fibrotic component selected from scleroderma, sarcodosis and systemic lupus erythematosus.

Thus, the present invention further relates to the use of the salts and crystal salt forms of the compound of above formula (I) for the preparation of a medicament for the treatment or prevention of the above-mentioned fibrotic diseases.

Within the meaning of the present invention, the treatment of the diseases may also be via simultaneous, separate or sequential co-administration of effective amounts of one or more salts and crystal salt forms of the compound of formula (I) in accordance with the present invention and at least a further chemotherapeutic or naturally occurring, semi-synthetic or synthetic therapeutic agent, in the form of a combined preparation, which if necessary may optionally be adapted for a co-treatment with radiotherapy or radio-immunotherapy.

The present invention also relates to a process for the treatment of the above-mentioned diseases, characterized in that one or more of the above-mentioned salts or crystal salt forms of the compound of formula (I) are administered in therapeutically effective amounts to a patient in need thereof

The present invention further relates to processes for the treatment of the aforementioned diseases, characterized in that one or more of the above-mentioned salts or crystal salt forms of the compound of formula (I) are administered once or several times a day or once or several times a week in therapeutically effective amounts.

The present invention also relates to pharmaceutical compositions comprising the above-mentioned salts or crystalline salt forms.

In the treatment of the aforementioned diseases, the physicochemical properties of a drug substance may influence decisively stability, usefulness and efficacy of the formulation. In this respect the salts or crystal salt forms of the compound of formula (I) in accordance with the present invention show advantageous properties not yet disclosed in the art.

Suitable preparations for the pharmaceutical compositions in accordance with the present invention include for example tablets, capsules, suppositories, solutions, - particularly solutions for injection (s.c., i.v., i.m.) and infusion - elixirs, emulsions or dispersible powders. The proportion of the pharmaceutically active compound(s) should be in the range from 0.01 to 90 wt.-%, preferably 0.1 to 50 wt.-% of the composition as a whole, i.e. in amounts which are sufficient to achieve the dosage necessary to achieve a therapeutic effect. If necessary the doses specified may be given several times a day.

Suitable tablets may be obtained, for example, by mixing the active substance(s) with known excipients, for example inert diluents such as calcium carbonate, calcium phosphate or lactose, disintegrants such as maize starch or alginic acid, binders such as starch or gelatine, lubricants such as magnesium stearate or talc and/or agents for delaying release, such as carboxymethyl cellulose, cellulose acetate phthalate, or polyvinyl acetate. The tablets may also comprise several layers.

Coated tablets may be prepared accordingly by coating cores produced analogously to the tablets with substances normally used for tablet coatings, for example collidone or shellac, gum arabic, talc, titanium dioxide or sugar. To achieve delayed release or prevent incompatibilities the core may also consist of a number of layers. Similarly the tablet coating may consist of a number or layers to achieve delayed release, possibly using the excipients mentioned above for the tablets.

Syrups or elixirs containing the active substances or combinations thereof according to the invention may additionally contain a sweetener such as saccharine, cyclamate, glycerol or sugar and a flavour enhancer, e.g. a flavouring such as vanillin or orange extract. They may also contain suspension adjuvants or thickeners such as sodium carboxymethyl cellulose, wetting agents such as, for example, condensation products of fatty alcohols with ethylene oxide, or preservatives such as p-hydroxybenzoates.

Solutions for injection and infusion are prepared in the usual way, e.g. with the addition of isotonic agents, preservatives such as p-hydroxybenzoates, or stabilisers such as alkali metal salts of ethylenediamine tetraacetic acid, optionally using emulsifiers and/or dispersants, whilst if water is used as the diluent, for example, organic solvents may optionally be used as solvating agents or dissolving aids, and transferred into injection vials or ampoules or infusion bottles.

Capsules containing one or more active substances or combinations of active substances may for example be prepared by mixing the active substances with inert carriers such as lactose or sorbitol and packing them into gelatine capsules.

Suitable suppositories may be made for example by mixing with carriers provided for this purpose, such as neutral fats or polyethyleneglycol or the derivatives thereof.

Excipients which may be used include, for example, water, pharmaceutically acceptable organic solvents such as paraffins (e.g. petroleum fractions), vegetable oils (e.g. groundnut or sesame oil), mono- or polyfunctional alcohols (e.g. ethanol or glycerol), carriers such as e.g. natural mineral powders (e.g. kaolins, clays, talc, chalk), synthetic mineral powders (e.g. highly dispersed silicic acid and silicates), sugars (e.g. cane sugar, lactose and glucose) emulsifiers (e.g. lignin, spent sulphite liquors, methylcellulose, starch and polyvinylpyrrolidone) and lubricants (e.g. magnesium stearate, talc, stearic acid and sodium lauryl sulphate).

The preparations are administered by the usual methods, preferably by oral route, by injection or transdermally. For oral administration the tablets may of course contain, apart from the abovementioned carriers, additives such as sodium citrate, calcium carbonate and dicalcium phosphate together with various additives such as starch, preferably potato starch, gelatine and the like. Moreover, lubricants such as magnesium stearate, sodium lauryl sulphate and talc may be used at the same time for the tabletting process. In the case of aqueous suspensions the active substances may be combined with various flavour enhancers or colourings in addition to the excipients mentioned above.

For parenteral use, solutions of the active substances with suitable liquid carriers may be used.

The dosage for intravenous use is from 1 - 1000 mg per hour, preferably between 5 and 500 mg per hour.

However, it may sometimes be necessary to depart from the amounts specified, depending on the body weight, the route of administration, the individual response to the drug, the nature of its formulation and the time or interval over which the drug is administered. Thus, in some cases it may be sufficient to use less than the minimum dose given above, whereas in other cases the upper limit may have to be exceeded. When administering large amounts it may be advisable to divide them up into a number of smaller doses spread over the day.

The following examples of pharmaceutical formulations illustrate the present invention without representing a limitation of its scope.

### 1. Coated tablet containing 75 mg of active substance

| Composition: 1 tablet contains | |
|---|---|
| active substance | 75.0 mg |
| calcium phosphate | 131.0 mg |
| polyvinylpyrrolidone | 10.0 mg |
| carboxymethylcellulose sodium | 10.0 mg |
| silicon dioxide | 2.5 mg |
| magnesium stearate | 1.5 mg |
| | 230.0 mg |

### Preparation (direct compression):

The active substance is mixed with all components, sieved and compressed in a tablet-making machine to form tablets of the desired shape.

| | |
|---|---|
| Weight of core: | 230 mg |
| Appearance of core: | 9 mm, biconvex |

The tablet cores thus produced are coated with a film consisting essentially of hydroxypropylmethylcellulose.

| | |
|---|---|
| Weight of coated tablet: | 240 mg. |

### 2. Tablet containing 100 mg of active substance

| Composition : 1 tablet contains | |
|---|---|
| active substance | 100.0 mg |
| lactose | 80.0 mg |
| corn starch | 34.0 mg |
| hydroxypropylmethylcellulose | 4.0 mg |
| magnesium stearate | 2.0 mg |
| | 220.0 mg |

### Preparation (wet granulation):

The active substance, lactose and starch are mixed together and uniformly moistened with an aqueous solution of the hydroxypropylmethylcellulose. After the moist composition has been screened (2.0 mm mesh size) and dried in a rack-type drier at 50°C it is screened again (1.5 mm mesh size) and the lubricant is added. The finished mixture is compressed to form tablets.

| | |
|---|---|
| Weight of tablet: | 220 mg |
| Appearance of tablet: | 10 mm, flat faced with bevelled edges and breaking notch on one side |

### 3. Tablet containing 150 mg of active substance

| Composition: 1 tablet contains | |
|---|---|
| active substance | 150.0 mg |
| lactose | 85.0 mg |
| microcrystalline cellulose | 40.0 mg |
| polyvinylpyrrolidone | 10.0 mg |
| silicon dioxide | 10.0 mg |
| magnesium stearate | 5.0 mg |
| | 300.0 mg |

### Preparation (dry granulation):

The active substance mixed with lactose, polyvinylpyrrolidone,and parts of the microcrystalline cellulose, magnesium stearate is compacted e.g. on a roller compactor. The ribbons are broken up in fine granules through a screen with a mesh size of 0.8 mm. After subsequent sieving through a screen with a mesh size of 0.5 mm and blending with the remaining components, tablets are pressed from the mixture.

| | |
|---|---|
| Weight of tablet: | 300 mg |
| Appearance of tablet: | 10 mm, flat |

### 4. Hard gelatine capsule containing 150 mg of active substance

| Composition: 1 capsule contains | |
|---|---|
| active substance | 150.0 mg |
| lactose | 85.0 mg |
| microcrystalline cellulose | 40.0 mg |
| polyvinylpyrrolidone | 10.0 mg |
| silicon dioxide | 10.0 mg |
| magnesium stearate | 5.0 mg |
| | 300.0 mg |

### Preparation:

The active substance mixed with lactose, polyvinylpyrrolidone,and parts of the microcrystalline cellulose, magnesium stearate is compacted e.g. on a roller compactor. The ribbons are broken up in fine granules through a screen with a mesh size of 0.8 mm. After subsequent sieving through a screen with a mesh size of 0.5 mm and blending with the remaining components, the finished mixture is packed into size 1 hard gelatine capsules.

| | |
|---|---|
| Capsule filling: | approx. 300 mg |
| Capsule shell: | size 1 hard gelatine capsule |

### 5. Suppository containing 150 mg of active substance

| 1 suppository contains: | |
|---|---|
| active substance | 150.0 mg |
| polyethyleneglycol 1500 | 800.0 mg |
| polyethyleneglycol 6000 | 850.0 mg |
| polyoxyl 40 hydrogenated castor oil | 200.0 mg |
| | 2,000.0 mg |

### Preparation:

After the suppository mass has been melted the active substance is homogeneously distributed therein and the melt is poured into chilled moulds.

### 6. Suspension containing 50 mg of active substance

| 100 ml of suspension contains: | |
|---|---|
| active substance | 1.00 g |
| carboxymethylcellulose sodium | 0.10 g |
| methyl p-hydroxybenzoate | 0.05 g |
| propyl p-hydroxybenzoate | 0.01 g |
| glucose | 10.00 g |
| glycerol | 5.00 g |
| 70% sorbitol solution | 20.00 g |
| flavouring | 0.30 g |
| dist. water ad | 100 ml |

### Preparation:

The distilled water is heated to 70°C. The methyl and propyl p-hydroxybenzoates together with the glycerol and sodium salt of carboxymethylcellulose are dissolved therein with stirring. The solution is cooled to ambient temperature and the active substance is added and homogeneously dispersed therein with stirring. After the sugar, the sorbitol solution and the flavouring have been added and dissolved, the suspension is evacuated with stirring to eliminate air.

Thus, 5 ml of suspension contains 50 mg of active substance.

### 7. Ampoule containing 10 mg active substance

| Composition: | |
|---|---|
| active substance | 10.0 mg |
| 0.01 N hydrochloric acid | q.s. |
| double-distilled water | ad 2.0 ml |

### Preparation:

The active substance is dissolved in the necessary amount of 0.01 N HCl, made isotonic with sodium chloride, filtered sterile and transferred into a 2 ml ampoule.

### 8. Ampoule containing 50 mg of active substance

| Composition: | |
|---|---|
| active substance | 50.0 mg |
| 0.01 N hydrochloric acid | q.s. |
| double-distilled water | ad 10.0 ml |

### Preparation:

The active substance is dissolved in the necessary amount of 0.01 N HCl, made isotonic with sodium chloride, filtered sterile and transferred into a 10 ml ampoule.

### 9. Capsule for powder inhalation containing 5 mg of active substance

| 1 capsule contains: | |
|---|---|
| active substance | 5.0 mg |
| lactose for inhalation | 15.0 mg |
| | 20.0 mg |

### Preparation:

The active substance is mixed with lactose for inhalation. The mixture is packed into capsules in a capsule-making machine (weight of the empty capsule approx. 50 mg).
Weight of capsule: 70.0 mg
Size of capsule = size 3

### 10. Solution for inhalation for a hand-held nebuliser containing 2.5 mg active substance

| 1 spray contains: | |
|---|---|
| active substance | 2.500 mg |
| benzalkonium chloride | 0.001 mg |
| IN hydrochloric acid q.s. ethanol/water (50/50) | ad 15.000 mg |

### Preparation:

The active substance and benzalkonium chloride are dissolved in ethanol/water (50/50). The pH of the solution is adjusted with IN hydrochloric acid. The resulting solution is filtered and transferred into suitable containers for use in hand-held nebulisers (cartridges).

Contents of the container: 4.5 g

## Claims

1. Salt of the compound 3-Z-[1-(4-(N-((4-methyl-piperazin-1-yl)-methylcarbonyl)-N-methyl-amino)-anilino)-1-phenyl-methylene]-6-methoxycarbonyl-2-indolinone, which is selected from the group consisting of:
the 3-Z-[1-(4-(N-((4-methyl-piperazin-1-yl)-methylcarbonyl)-N-methyl-amino)-anilino)-1-phenyl-methylene]-6-methoxycarbonyl-2-indolinonechloride;
the 3-Z-[1-(4-(N-((4-methyl-piperazin-1-yl)-methylcarbonyl)-N-methyl-amino)-anilino)-1-phenyl-methylene]-6-methoxycarbonyl-2-indolinone bromide;
the 3-Z-[1-(4-(N-((4-methyl-piperazin-1-yl)-methylcarbonyl)-N-methyl-amino)-anilino)-1-phenyl-methylene]-6-methoxycarbonyl-2-indolinone phosphate;
the 3-Z-[1-(4-(N-((4-methyl-piperazin-1-yl)-methylcarbonyl)-N-methyl-amino)-anilino)-1-phenyl-methylene]-6-methoxycarbonyl-2-indolinone sulfate;
the 3-Z-[1-(4-(N-((4-methyl-piperazin-1-yl)-methylcarbonyl)-N-methyl-amino)-anilino)-1-phenyl-methylene]-6-methoxycarbonyl-2-indolinone methanesulfonate;
the 3-Z-[1-(4-(N-((4-methyl-piperazin-1-yl)-methylcarbonyl)-N-methyl-amino)-anilino)-1-phenyl-methylene]-6-methoxycarbonyl-2-indolinone ethanedisulfonate;
the bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}ethanedisulfonate;
the 3-Z-[1-(4-(N-((4-methyl-piperazin-1-yl)-methylcarbonyl)-N-methyl-amino)-anilino)-1-phenyl-methylene]-6-methoxycarbonyl-2-indolinone isethionate;
the 3-Z-[1-(4-(N-((4-methyl-piperazin-1-yl)-methylcarbonyl)-N-methyl-amino)-anilino)-1-phenyl-methylene]-6-methoxycarbonyl-2-indolinonebenzenesulfonate;
the 3-Z-[1-(4-(N-((4-methyl-piperazin-1-yl)-methylcarbonyl)-N-methyl-amino)-anilino)-1-phenyl-methylene]-6-methoxycarbonyl-2-indolinone tosylate;
the 3-Z-[1-(4-(N-((4-methyl-piperazin-1-yl)-methylcarbonyl)-N-methyl-amino)-anilino)-1-phenyl-methylene]-6-methoxycarbonyl-2-indolinone camphorsulfonate;
the 3-Z-[1-(4-(N-((4-methyl-piperazin-1-yl)-methylcarbonyl)-N-methyl-amino)-anilino)-1-phenyl-methylene]-6-methoxycarbonyl-2-indolinone naphthalene-1,5-disulfonate;
the 3-Z-[1-(4-(N-((4-methyl-piperazin-1-yl)-methylcarbonyl)-N-methyl-amino)-anilino)-1-phenyl-methylene]-6-methoxycarbonyl-2-indolinone citrate;
the bis{3-Z-[1-(4-(N-((4-methyl-piperazin-1-yl)-methylcarbonyl)-N-methyl-amino)-anilino)-1-phenyl-methylene] -6-methoxycarbonyl-2-indolinone}citrate;
the 3-Z-[1-(4-(N-((4-methyl-piperazin-1-yl)-methylcarbonyl)-N-methyl-amino)-anilino)-1-phenyl-methylene]-6-methoxycarbonyl-2-indolinone D-tartrate;
the bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone} D-tartrate;
the 3-Z-[1-(4-(N-((4-methyl-piperazin-1-yl)-methylcarbonyl)-N-methyl-amino)-anilino)-1-phenyl-methylene]-6-methoxycarbonyl-2-indolinone L-tartrate;
the bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone} L-tartrate;
the bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone} fumarate;
the bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}maleate;
the 3-Z-[1-(4-(N-((4-methyl-piperazin-1-yl)-methylcarbonyl)-N-methyl-amino)-anilino)-1-phenyl-methylene]-6-methoxycarbonyl-2-indolinone L-lactate;
the 3-Z-[1-(4-(N-((4-methyl-piperazin-1-yl)-methylcarbonyl)-N-methyl-amino)-anilino)-1-phenyl-methylene]-6-methoxycarbonyl-2-indolinone glycolate;
the 3-Z-[1-(4-(N-((4-methyl-piperazin-1-yl)-methylcarbonyl)-N-methyl-amino)-anilino)-1-phenyl-methylene]-6-methoxycarbonyl-2-indolinone glycinate;
the 3-Z-[1-(4-(N-((4-methyl-piperazin-1-yl)-methylcarbonyl)-N-methyl-amino)-anilino)-1-phenyl-methylene]-6-methoxycarbonyl-2-indolinone L-malate;
the bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone} L-malate;
the 3-Z-[1-(4-(N-((4-methyl-piperazin-1-yl)-methylcarbonyl)-N-methyl-amino)-anilino)-1-phenyl-methylene]-6-methoxycarbonyl-2-indolinone D-malate;
the bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}D-malate;
the 3-Z-[1-(4-(N-((4-methyl-piperazin-1-yl)-methylcarbonyl)-N-methyl-amino)-anilino)-1-phenyl-methylene]-6-methoxycarbonyl-2-indolinone malonate;
the bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}malonate;
the bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone} succinate;
the 3-Z-[1-(4-(N-((4-methyl-piperazin-1-yl)-methylcarbonyl)-N-methyl-amino)-anilino)-1-phenyl-methylene]-6-methoxycarbonyl-2-indolinone oxalate;
the bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone} oxalate;
the 3-Z-[1-(4-(N-((4-methyl-piperazin-1-yl)-methylcarbonyl)-N-methyl-amino)-anilino)-1-phenyl-methylene]-6-methoxycarbonyl-2-indolinone gentisate;
the 3-Z-[1-(4-(N-((4-methyl-piperazin-1-yl)-methylcarbonyl)-N-methyl-amino)-anilino)-1-phenyl-methylene]-6-methoxycarbonyl-2-indolinone camphorate;
the 3-Z-[1-(4-(N-((4-methyl-piperazin-1-yl)-methylcarbonyl)-N-methyl-amino)-anilino)-1-phenyl-methylene]-6-methoxycarbonyl-2-indolinone benzoate;
the 3-Z-[1-(4-(N-((4-methyl-piperazin-1-yl)-methylcarbonyl)-N-methyl-amino)-anilino)-1-phenyl-methylene]-6-methoxycarbonyl-2-indolinone mandelate;
the 3-Z-[1-(4-(N-((4-methyl-piperazin-1-yl)-methylcarbonyl)-N-methyl-amino)-anilino)-1-phenyl-methylene]-6-methoxycarbonyl-2-indolinone saccharinate;
the 3-Z-[1-(4-(N-((4-methyl-piperazin-1-yl)-methylcarbonyl)-N-methyl-amino)-anilino)-1-phenyl-methylene]-6-methoxycarbonyl-2-indolinonesalicylate;
the bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}L-aspartate;
the 3-Z-[1-(4-(N-((4-methyl-piperazin-1-yl)-methylcarbonyl)-N-methyl-amino)-anilino)-1-phenyl-methylene]-6-methoxycarbonyl-2-indolinone xinafoate; and
the 3-Z-[1-(4-(N-((4-methyl-piperazin-1-yl)-methylcarbonyl)-N-methyl-amino)-anilino)-1-phenyl-methylene]-6-methoxycarbonyl-2-indolinone ascorbate.

2. Crystalline salt form of the compound 3-Z-[1-(4-(N-((4-methyl-piperazin-1-yl)-methylcarbonyl)-N-methyl-amino)-anilino)-1-phenyl-methylene]-6-methoxycarbonyl-2-indolinone, which is selected from the group consisting of:
crystalline anhydrous 3-Z-[1-(4-(N-((4-methyl-piperazin-1-yl)-methylcarbonyl)-N-methyl-amino)-anilino)-1-phenyl-methylene]-6-methoxycarbonyl-2-indolinone chloride;
crystalline hydrated 3-Z-[1-(4-(N-((4-methyl-piperazin-1-yl)-methylcarbonyl)-N-methyl-amino)-anilino)-1-phenyl-methylene]-6-methoxycarbonyl-2-indolinone chloride;
crystalline hydrated 3-Z-[1-(4-(N-((4-methyl-piperazin-1-yl)-methylcarbonyl)-N-methyl-amino)-anilino)-1-phenyl-methylene]-6-methoxycarbonyl-2-indolinone bromide;
crystalline anhydrous 3-Z-[1-(4-(N-((4-methyl-piperazin-1-yl)-methylcarbonyl)-N-methyl-amino)-anilino)-1-phenyl-methylene]-6-methoxycarbonyl-2-indolinone bromide;
crystalline 3-Z-[1-(4-(N-((4-methyl-piperazin-1-yl)-methylcarbonyl)-N-methyl-amino)-anilino)-1-phenyl-methylene]-6-methoxycarbonyl-2-indolinone phosphate;
crystalline hydrated 3-Z-[1-(4-(N-((4-methyl-piperazin-1-yl)-methylcarbonyl)-N-methyl-amino)-anilino)-1-phenyl-methylene]-6-methoxycarbonyl-2-indolinone phosphate;
crystalline hydrated 3-Z-[1-(4-(N-((4-methyl-piperazin-1-yl)-methylcarbonyl)-N-methyl-amino)-anilino)-1-phenyl-methylene]-6-methoxycarbonyl-2-indolinone sulfate;
crystalline 3-Z-[1-(4-(N-((4-methyl-piperazin-1-yl)-methylcarbonyl)-N-methyl-amino)-anilino)-1-phenyl-methylene]-6-methoxycarbonyl-2-indolinone sulfate;
crystalline hydrated 3-Z-[1-(4-(N-((4-methyl-piperazin-1-yl)-methylcarbonyl)-N-methyl-amino)-anilino)-1-phenyl-methylene]-6-methoxycarbonyl-2-indolinone methanesulfonate;
crystalline 3-Z-[1-(4-(N-((4-methyl-piperazin-1-yl)-methylcarbonyl)-N-methyl-amino)-anilino)-1-phenyl-methylene]-6-methoxycarbonyl-2-indolinone ethanedisulfonate;
crystalline bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone} ethanedisulfonate;
crystalline hydrated 3-Z-[1-(4-(N-((4-methyl-piperazin-1-yl)-methylcarbonyl)-N-methyl-amino)-anilino)-1-phenyl-methylene]-6-methoxycarbonyl-2-indolinone ethanedisulfonate;
crystalline hydrated bis{3-Z-[1-(4-(N-((4-methyl-piperazin-1-yl)-methylcarbonyl)-N-methyl-amino)-anilino)-1-phenyl-methylene] -6-methoxycarbonyl-2-indolinone} ethanedisulfonate;
crystalline anhydrous 3-Z-[1-(4-(N-((4-methyl-piperazin-1-yl)-methylcarbonyl)-N-methyl-amino)-anilino)-1-phenyl-methylene]-6-methoxycarbonyl-2-indolinone isethionate;
crystalline 3-Z-[1-(4-(N-((4-methyl-piperazin-1-yl)-methylcarbonyl)-N-methyl-amino)-anilino)-1-phenyl-methylene]-6-methoxycarbonyl-2-indolinone isethionate;
crystalline hydrated 3-Z-[1-(4-(N-((4-methyl-piperazin-1-yl)-methylcarbonyl)-N-methyl-amino)-anilino)-1-phenyl-methylene]-6-methoxycarbonyl-2-indolinone isethionate;
crystalline hydrated 3-Z-[1-(4-(N-((4-methyl-piperazin-1-yl)-methylcarbonyl)-N-methyl-amino)-anilino)-1-phenyl-methylene]-6-methoxycarbonyl-2-indolinone benzenesulfonate;
crystalline 3-Z-[1-(4-(N-((4-methyl-piperazin-1-yl)-methylcarbonyl)-N-methyl-amino)-anilino)-1-phenyl-methylene]-6-methoxycarbonyl-2-indolinone benzenesulfonate;
crystalline hydrated 3-Z-[1-(4-(N-((4-methyl-piperazin-1-yl)-methylcarbonyl)-N-methyl-amino)-anilino)-1-phenyl-methylene]-6-methoxycarbonyl-2-indolinone tosylate;
crystalline anhydrous 3-Z-[1-(4-(N-((4-methyl-piperazin-1-yl)-methylcarbonyl)-N-methyl-amino)-anilino)-1-phenyl-methylene]-6-methoxycarbonyl-2-indolinone camphorsulfonate;
crystalline solvated 3-Z-[1-(4-(N-((4-methyl-piperazin-1-yl)-methylcarbonyl)-N-methyl-amino)-anilino)-1-phenyl-methylene]-6-methoxycarbonyl-2-indolinone camphorsulfonate;
crystalline 3-Z-[1-(4-(N-((4-methyl-piperazin-1-yl)-methylcarbonyl)-N-methyl-amino)-anilino)-1-phenyl-methylene]-6-methoxycarbonyl-2-indolinone naphthalene-1,5-disulfonate;
crystalline hydrated 3-Z-[1-(4-(N-((4-methyl-piperazin-1-yl)-methylcarbonyl)-N-methyl-amino)-anilino)-1-phenyl-methylene]-6-methoxycarbonyl-2-indolinone naphthalene-1, 5-disulfonate;
crystalline anhydrous 3-Z-[1-(4-(N-((4-methyl-piperazin-1-yl)-methylcarbonyl)-N-methyl-amino)-anilino)-1-phenyl-methylene]-6-methoxycarbonyl-2-indolinone citrate;
crystalline hydrated bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}citrate;
crystalline hydrated 3-Z-[1-(4-(N-((4-methyl-piperazin-1-yl)-methylcarbonyl)-N-methyl-amino)-anilino)-1-phenyl-methylene]-6-methoxycarbonyl-2-indolinone D-tartrate;
crystalline bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}D-tartrate;
crystalline hydrated 3-Z-[1-(4-(N-((4-methyl-piperazin-1-yl)-methylcarbonyl)-N-methyl-amino)-anilino)-1-phenyl-methylene]-6-methoxycarbonyl-2-indolinone L-tartrate;
crystalline bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}L-tartrate;
crystalline hydrated bis{3-Z-[1-(4-(N-((4-methyl-piperazin-1-yl)-methylcarbonyl)-N-methyl-amino)-anilino)-1-phenyl-methylene] -6-methoxycarbonyl-2-indolinone} fumarate;
crystalline anhydrous bis{3-Z-[1-(4-(N-((4-methyl-piperazin-1-yl)-methylcarbonyl)-N-methyl-amino)-anilino)-1-phenyl-methylene] -6-methoxycarbonyl-2-indolinone} fumarate;
crystalline anhydrous bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}maleate;
crystalline hydrated 3-Z-[1-(4-(N-((4-methyl-piperazin-1-yl)-methylcarbonyl)-N-methyl-amino)-anilino)-1-phenyl-methylene]-6-methoxycarbonyl-2-indolinone L-lactate;
crystalline hydrated 3-Z-[1-(4-(N-((4-methyl-piperazin-1-yl)-methylcarbonyl)-N-methyl-amino)-anilino)-1-phenyl-methylene]-6-methoxycarbonyl-2-indolinone glycolate;
crystalline 3-Z-[1-(4-(N-((4-methyl-piperazin-1-yl)-methylcarbonyl)-N-methyl-amino)-anilino)-1-phenyl-methylene]-6-methoxycarbonyl-2-indolinone glycinate;
crystalline hydrated 3-Z-[1-(4-(N-((4-methyl-piperazin-1-yl)-methylcarbonyl)-N-methyl-amino)-anilino)-1-phenyl-methylene]-6-methoxycarbonyl-2-indolinone L-malate;
crystalline 3-Z-[1-(4-(N-((4-methyl-piperazin-1-yl)-methylcarbonyl)-N-methyl-amino)-anilino)-1-phenyl-methylene]-6-methoxycarbonyl-2-indolinone L-malate;
crystalline bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}L-malate;
crystalline hydrated bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}L-malate;
crystalline hydrated 3-Z-[1-(4-(N-((4-methyl-piperazin-1-yl)-methylcarbonyl)-N-methyl-amino)-anilino)-1-phenyl-methylene]-6-methoxycarbonyl-2-indolinone D-malate;
crystalline bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}D-malate;
crystalline hydrated 3-Z-[1-(4-(N-((4-methyl-piperazin-1-yl)-methylcarbonyl)-N-methyl-amino)-anilino)-1-phenyl-methylene]-6-methoxycarbonyl-2-indolinone malonate;
crystalline hydrated bis{3-Z-[1-(4-(N-((4-methyl-piperazin-1-yl)-methylcarbonyl)-N-methyl-amino)-anilino)-1-phenyl-methylene] -6-methoxycarbonyl-2-indolinone}malonate;
crystalline solvated bis{3-Z-[1-(4-(N-((4-methyl-piperazin-1-yl)-methylcarbonyl)-N-methyl-amino)-anilino)- I -phenyl-methylene] -6-methoxycarbonyl-2-indolinone}malonate;
crystalline bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}malonate;
crystalline hydrated bis{3-Z-[1-(4-(N-((4-methyl-piperazin-1-yl)-methylcarbonyl)-N-methyl-amino)-anilino)-1-phenyl-methylene] -6-methoxycarbonyl-2-indolinone} succinate;
crystalline hydrated 3-Z-[1-(4-(N-((4-methyl-piperazin-1-yl)-methylcarbonyl)-N-methyl-amino)-anilino)-1-phenyl-methylene]-6-methoxycarbonyl-2-indolinone oxalate;
crystalline hydrated bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone} oxalate;
crystalline 3-Z-[1-(4-(N-((4-methyl-piperazin-1-yl)-methylcarbonyl)-N-methyl-amino)-anilino)-1-phenyl-methylene]-6-methoxycarbonyl-2-indolinone oxalate;
crystalline bis{3-Z-[1-(4-(N-((4-methyl-piperazin-l-yl)-methylcarbonyl)-N-methyl-amino)-anilino)-1-phenyl-methylene]-6-methoxycarbonyl-2-indolinone}oxalate;
crystalline hydrated bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}oxalate;
crystalline hydrated 3-Z-[1-(4-(N-((4-methyl-piperazin-1-yl)-methylcarbonyl)-N-methyl-amino)-anilino)-1-phenyl-methylene]-6-methoxycarbonyl-2-indolinone gentisate;
crystalline hydrated 3-Z-[1-(4-(N-((4-methyl-piperazin-1-yl)-methylcarbonyl)-N-methyl-amino)-anilino)-1-phenyl-methylene]-6-methoxycarbonyl-2-indolinone camphorate;
crystalline 3-Z-[1-(4-(N-((4-methyl-piperazin-1-yl)-methylcarbonyl)-N-methyl-amino)-anilino)-1-phenyl-methylene]-6-methoxycarbonyl-2-indolinone camphorate;
crystalline hydrated 3-Z-[1-(4-(N-((4-methyl-piperazin-1-yl)-methylcarbonyl)-N-methyl-amino)-anilino)-1-phenyl-methylene]-6-methoxycarbonyl-2-indolinone benzoate;
crystalline 3-Z-[1-(4-(N-((4-methyl-piperazin-1-yl)-methylcarbonyl)-N-methyl-amino)-anilino)-1-phenyl-methylene]-6-methoxycarbonyl-2-indolinone benzoate;
crystalline hydrated 3-Z-[1-(4-(N-((4-methyl-piperazin-1-yl)-methylcarbonyl)-N-methyl-amino)-anilino)-1-phenyl-methylene]-6-methoxycarbonyl-2-indolinone mandelate;
crystalline hydrated 3-Z-[1-(4-(N-((4-methyl-piperazin-1-yl)-methylcarbonyl)-N-methyl-amino)-anilino)-1-phenyl-methylene]-6-methoxycarbonyl-2-indolinone saccharinate;
crystalline 3-Z-[1-(4-(N-((4-methyl-piperazin-1-yl)-methylcarbonyl)-N-methyl-amino)-anilino)-1-phenyl-methylene]-6-methoxycarbonyl-2-indolinone saccharinate;
crystalline 3-Z-[1-(4-(N-((4-methyl-piperazin-1-yl)-methylcarbonyl)-N-methyl-amino)-anilino)-1-phenyl-methylene]-6-methoxycarbonyl-2-indolinone salicylate;
crystalline anhydrous 3-Z-[1-(4-(N-((4-methyl-piperazin-1-yl)-methylcarbonyl)-N-methyl-amino)-anilino)-1-phenyl-methylene]-6-methoxycarbonyl-2-indolinone salicylate;
crystalline hydrated bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}L-aspartate;
crystalline bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}L-aspartate;
crystalline hydrated 3-Z-[1-(4-(N-((4-methyl-piperazin-1-yl)-methylcarbonyl)-N-methyl-amino)-anilino)-1-phenyl-methylene]-6-methoxycarbonyl-2-indolinone xinafoate; and
crystalline 3-Z-[1-(4-(N-((4-methyl-piperazin-1-yl)-methylcarbonyl)-N-methyl-amino)-anilino)-1-phenyl-methylene]-6-methoxycarbonyl-2-indolinone ascorbate.

3. Crystalline salt form of the compound 3-Z-[1-(4-(N-((4-methyl-piperazin-1-yl)-methylcarbonyl)-N-methyl-amino)-anilino)-1-phenyl-methylene]-6-methoxycarbonyl-2-indolinone in accordance with claim 2, which is selected from the group consisting of:
3-Z-[1-(4-(N-((4-methyl-piperazin-1-yl)-methylcarbonyl)-N-methyl-amino)-anilino)-1-phenyl-methylene]-6-methoxycarbonyl-2-indolinone chloride in crystalline anhydrous Form I;
3-Z-[1-(4-(N-((4-methyl-piperazin-1-yl)-methylcarbonyl)-N-methyl-amino)-anilino)-1-phenyl-methylene]-6-methoxycarbonyl-2-indolinone chloride in crystalline hydrated Form II;
3-Z-[1-(4-(N-((4-methyl-piperazin-1-yl)-methylcarbonyl)-N-methyl-amino)-anilino)-1-phenyl-methylene]-6-methoxycarbonyl-2-indolinone bromide in crystalline trihydrated Form II;
3-Z-[1-(4-(N-((4-methyl-piperazin-1-yl)-methylcarbonyl)-N-methyl-amino)-anilino)-1-phenyl-methylene]-6-methoxycarbonyl-2-indolinone bromide in crystalline anhydrous Form VII;
3-Z-[1-(4-(N-((4-methyl-piperazin-1-yl)-methylcarbonyl)-N-methyl-amino)-anilino)-1-phenyl-methylene]-6-methoxycarbonyl-2-indolinone bromide in crystalline anhydrous Form VIII;
3-Z-[1-(4-(N-((4-methyl-piperazin-1-yl)-methylcarbonyl)-N-methyl-amino)-anilino)-1-phenyl-methylene]-6-methoxycarbonyl-2-indolinone phosphate in crystalline Form I;
3-Z-[1-(4-(N-((4-methyl-piperazin-1-yl)-methylcarbonyl)-N-methyl-amino)-anilino)-1-phenyl-methylene]-6-methoxycarbonyl-2-indolinone phosphate in crystalline 3,5-hydrated Form II;
3-Z-[1-(4-(N-((4-methyl-piperazin-1-yl)-methylcarbonyl)-N-methyl-amino)-anilino)-1-phenyl-methylene]-6-methoxycarbonyl-2-indolinone sulfate in crystalline hydrated Form I;
3-Z-[1-(4-(N-((4-methyl-piperazin-1-yl)-methylcarbonyl)-N-methyl-amino)-anilino)-1-phenyl-methylene]-6-methoxycarbonyl-2-indolinone sulfate in crystalline Form V;
3-Z-[1-(4-(N-((4-methyl-piperazin-1-yl)-methylcarbonyl)-N-methyl-amino)-anilino)-1-phenyl-methylene]-6-methoxycarbonyl-2-indolinone sulfate in crystalline hydrated Form VI;
3-Z-[1-(4-(N-((4-methyl-piperazin-1-yl)-methylcarbonyl)-N-methyl-amino)-anilino)-1-phenyl-methylene]-6-methoxycarbonyl-2-indolinone sulfate in crystalline hydrated Form VII;
3-Z-[1-(4-(N-((4-methyl-piperazin-1-yl)-methylcarbonyl)-N-methyl-amino)-anilino)-1-phenyl-methylene]-6-methoxycarbonyl-2-indolinone methanesulfonate in crystalline hemihydrated Form I;
3-Z-[1-(4-(N-((4-methyl-piperazin-1-yl)-methylcarbonyl)-N-methyl-amino)-anilino)-1-phenyl-methylene]-6-methoxycarbonyl-2-indolinone ethanedisulfonate in crystalline Form I;
bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}ethanedisulfonate in crystalline Form I;
3-Z-[1-(4-(N-((4-methyl-piperazin-1-yl)-methylcarbonyl)-N-methyl-amino)-anilino)-1-phenyl-methylene]-6-methoxycarbonyl-2-indolinone ethanedisulfonate in crystalline Form II;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}ethanedisulfonate in crystalline Form II;
3-Z-[1-(4-(N-((4-methyl-piperazin-1-yl)-methylcarbonyl)-N-methyl-amino)-anilino)-1-phenyl-methylene]-6-methoxycarbonyl-2-indolinone ethanedisulfonate in crystalline hydrated Form III;
Bis{3-Z-[1-(4-(N-((4-methyl-piperazin-1-yl)-methylcarbonyl)-N-methyl-amino)-anilino)-1-phenyl- methylene]-6-methoxycarbonyl-2-indolinone}ethanedisulfonate in crystalline hydrated Form III;
3-Z-[1-(4-(N-((4-methyl-piperazin-1-yl)-methylcarbonyl)-N-methyl-amino)-anilino)-1-phenyl-methylene]-6-methoxycarbonyl-2-indolinone ethanedisulfonate in crystalline Form V;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}ethanedisulfonate in crystalline Form V;
3-Z-[1-(4-(N-((4-methyl-piperazin-1-yl)-methylcarbonyl)-N-methyl-amino)-anilino)-1-phenyl-methylene]-6-methoxycarbonyl-2-indolinone ethanedisulfonate in crystalline Form VI;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}ethanedisulfonate in crystalline Form VI;
3-Z-[1-(4-(N-((4-methyl-piperazin-1-yl)-methylcarbonyl)-N-methyl-amino)-anilino)-1-phenyl-methylene]-6-methoxycarbonyl-2-indolinone ethanedisulfonate in crystalline anhydrous Form VII;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}ethanedisulfonate in crystalline anhydrous Form VII;
3-Z-[1-(4-(N-((4-methyl-piperazin-1-yl)-methylcarbonyl)-N-methyl-amino)-anilino)-1-phenyl-methylene]-6-methoxycarbonyl-2-indolinone isethionate in crystalline anhydrous Form I;
3-Z-[1-(4-(N-((4-methyl-piperazin-1-yl)-methylcarbonyl)-N-methyl-amino)-anilino)-1-phenyl-methylene]-6-methoxycarbonyl-2-indolinone isethionate in crystalline Form II;
3-Z-[1-(4-(N-((4-methyl-piperazin-1-yl)-methylcarbonyl)-N-methyl-amino)-anilino)-1-phenyl-methylene]-6-methoxycarbonyl-2-indolinone isethionate in crystalline Form IV;
3-Z-[1-(4-(N-((4-methyl-piperazin-1-yl)-methylcarbonyl)-N-methyl-amino)-anilino)-1-phenyl-methylene]-6-methoxycarbonyl-2-indolinone isethionate in crystalline dihydrated Form V;
3-Z-[1-(4-(N-((4-methyl-piperazin-1-yl)-methylcarbonyl)-N-methyl-amino)-anilino)-1-phenyl-methylene]-6-methoxycarbonyl-2-indolinone benzenesulfonate in crystalline trihydrated Form I;
3-Z-[1-(4-(N-((4-methyl-piperazin-1-yl)-methylcarbonyl)-N-methyl-amino)-anilino)-1-phenyl-methylene]-6-methoxycarbonyl-2-indolinone benzenesulfonate in crystalline Form III;
3-Z-[1-(4-(N-((4-methyl-piperazin-1-yl)-methylcarbonyl)-N-methyl-amino)-anilino)-1-phenyl-methylene]-6-methoxycarbonyl-2-indolinone benzenesulfonate in crystalline Form V;
3-Z-[1-(4-(N-((4-methyl-piperazin-1-yl)-methylcarbonyl)-N-methyl-amino)-anilino)-1-phenyl-methylene]-6-methoxycarbonyl-2-indolinone tosylate in crystalline monohydrated Form I;
3-Z-[1-(4-(N-((4-methyl-piperazin-1-yl)-methylcarbonyl)-N-methyl-amino)-anilino)-1-phenyl-methylene]-6-methoxycarbonyl-2-indolinone tosylate in crystalline trihydrated Form II;
3-Z-[1-(4-(N-((4-methyl-piperazin-1-yl)-methylcarbonyl)-N-methyl-amino)-anilino)-1-phenyl-methylene]-6-methoxycarbonyl-2-indolinone camphorsulfonate in crystalline anhydrate Form II;
3-Z-[1-(4-(N-((4-methyl-piperazin-1-yl)-methylcarbonyl)-N-methyl-amino)-anilino)-1-phenyl-methylene]-6-methoxycarbonyl-2-indolinone camphorsulfonate in crystalline anhydrous Form III;
3-Z-[1-(4-(N-((4-methyl-piperazin-1-yl)-methylcarbonyl)-N-methyl-amino)-anilino)-1-phenyl-methylene]-6-methoxycarbonyl-2-indolinone camphorsulfonate in crystalline hemisolvated Form V with propionitrile;
3-Z-[1-(4-(N-((4-methyl-piperazin-1-yl)-methylcarbonyl)-N-methyl-amino)-anilino)-1-phenyl-methylene]-6-methoxycarbonyl-2-indolinone camphorsulfonate in hemisolvated Form XII with 1,2-dimethoxyethane;
3-Z-[1-(4-(N-((4-methyl-piperazin-1-yl)-methylcarbonyl)-N-methyl-amino)-anilino)-1-phenyl-methylene]-6-methoxycarbonyl-2-indolinone naphthalene-1,5-disulfonate in crystalline Form III;
3-Z-[1-(4-(N-((4-methyl-piperazin-1-yl)-methylcarbonyl)-N-methyl-amino)-anilino)-1-phenyl-methylene]-6-methoxycarbonyl-2-indolinone naphthalene-1,5-disulfonate in crystalline Form IV;
3-Z-[1-(4-(N-((4-methyl-piperazin-1-yl)-methylcarbonyl)-N-methyl-amino)-anilino)-1-phenyl-methylene]-6-methoxycarbonyl-2-indolinone naphthalene-1,5-disulfonate in crystalline hydrated Form V;
3-Z-[1-(4-(N-((4-methyl-piperazin-1-yl)-methylcarbonyl)-N-methyl-amino)-anilino)-1-phenyl-methylene]-6-methoxycarbonyl-2-indolinone citrate in crystalline anhydrous Form I;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}citrate in crystalline dihydrated Form II;
3-Z-[1-(4-(N-((4-methyl-piperazin-1-yl)-methylcarbonyl)-N-methyl-amino)-anilino)-1-phenyl-methylene]-6-methoxycarbonyl-2-indolinone D-tartrate in crystalline hydrated Form I;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}D-tartrate in crystalline Form II;
3-Z-[1-(4-(N-((4-methyl-piperazin-1-yl)-methylcarbonyl)-N-methyl-amino)-anilino)-1-phenyl-methylene]-6-methoxycarbonyl-2-indolinone L-tartrate in crystalline hydrated Form I;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone} L-tartrate in crystalline Form II;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}fumarate in crystalline hydrated Form I;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}fumarate in crystalline anhydrous Form III;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}maleate in crystalline anhydrous Form I;
3-Z-[1-(4-(N-((4-methyl-piperazin-1-yl)-methylcarbonyl)-N-methyl-amino)-anilino)-1-phenyl-methylene]-6-methoxycarbonyl-2-indolinone L-lactate in crystalline 2,5-hydrated Form I;
3-Z-[1-(4-(N-((4-methyl-piperazin-1-yl)-methylcarbonyl)-N-methyl-amino)-anilino)-1-phenyl-methylene]-6-methoxycarbonyl-2-indolinone glycolate in crystalline hydrated Form I;
3-Z-[1-(4-(N-((4-methyl-piperazin-1-yl)-methylcarbonyl)-N-methyl-amino)-anilino)-1-phenyl-methylene]-6-methoxycarbonyl-2-indolinone glycinate in crystalline Form I;
3-Z-[1-(4-(N-((4-methyl-piperazin-1-yl)-methylcarbonyl)-N-methyl-amino)-anilino)-1-phenyl-methylene]-6-methoxycarbonyl-2-indolinone L-malate in crystalline hydrated Form I;
3-Z-[1-(4-(N-((4-methyl-piperazin-1-yl)-methylcarbonyl)-N-methyl-amino)-anilino)-1-phenyl-methylene]-6-methoxycarbonyl-2-indolinone L-malate in crystalline Form II;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}L-malate in crystalline Form II;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}L-malate in crystalline tetrahydrated Form III;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}L-malate in crystalline Form IV;
3-Z-[1-(4-(N-((4-methyl-piperazin-1-yl)-methylcarbonyl)-N-methyl-amino)-anilino)-1-phenyl-methylene]-6-methoxycarbonyl-2-indolinone D-malate in crystalline hydrated Form I;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}D-malate in crystalline Form IV;
3-Z-[1-(4-(N-((4-methyl-piperazin-1-yl)-methylcarbonyl)-N-methyl-amino)-anilino)-1-phenyl-methylene]-6-methoxycarbonyl-2-indolinone malonate in crystalline hydrated Form I;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}malonate in crystalline hydrated Form I;
3-Z-[1-(4-(N-((4-methyl-piperazin-1-yl)-methylcarbonyl)-N-methyl-amino)-anilino)-1-phenyl-methylene]-6-methoxycarbonyl-2-indolinone malonate in crystalline hydrated Form II;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}malonate in crystalline hydrated Form II;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}malonate in crystalline hydrated and/or solvated Form III;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}malonate in crystalline Form IV;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}malonate in crystalline Form V;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}malonate in crystalline Form VI;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}succinate in crystalline hydrated Form I;
Bis {3-Z-[1-(4-(N-((4-methyl-piperazin-1-yl)-methylcarbonyl)-N-methyl-amino)-anilino)-1-phenyl-methylene]-6-methoxycarbonyl-2-indolinone}succinate in crystalline hydrated Form II;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}succinate in crystalline hexahydrated Form III;
3-Z-[1-(4-(N-((4-methyl-piperazin-1-yl)-methylcarbonyl)-N-methyl-amino)-anilino)-1-phenyl-methylene]-6-methoxycarbonyl-2-indolinone oxalate in crystalline hydrated Form III;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}oxalate in crystalline hydrated Form III;
3-Z-[1-(4-(N-((4-methyl-piperazin-1-yl)-methylcarbonyl)-N-methyl-amino)-anilino)-1-phenyl-methylene]-6-methoxycarbonyl-2-indolinone oxalate in crystalline Form V;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}oxalate in crystalline Form V;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}oxalate in crystalline hydrated Form VI;
3-Z-[1-(4-(N-((4-methyl-piperazin-1-yl)-methylcarbonyl)-N-methyl-amino)-anilino)-1-phenyl-methylene]-6-methoxycarbonyl-2-indolinone gentisate in crystalline hydrated Form I;
3-Z-[1-(4-(N-((4-methyl-piperazin-1-yl)-methylcarbonyl)-N-methyl-amino)-anilino)-1-phenyl-methylene]-6-methoxycarbonyl-2-indolinone gentisate in crystalline hemihydrated Form XI;
3-Z-[1-(4-(N-((4-methyl-piperazin-1-yl)-methylcarbonyl)-N-methyl-amino)-anilino)-1-phenyl-methylene]-6-methoxycarbonyl-2-indolinone camphorate in crystalline hydrated Form II;
3-Z-[1-(4-(N-((4-methyl-piperazin-1-yl)-methylcarbonyl)-N-methyl-amino)-anilino)-1-phenyl-methylene]-6-methoxycarbonyl-2-indolinone camphorate in crystalline Form III;
3-Z-[1-(4-(N-((4-methyl-piperazin-1-yl)-methylcarbonyl)-N-methyl-amino)-anilino)-1-phenyl-methylene]-6-methoxycarbonyl-2-indolinone benzoate in crystalline hydrated Form II;
3-Z-[1-(4-(N-((4-methyl-piperazin-1-yl)-methylcarbonyl)-N-methyl-amino)-anilino)-1-phenyl-methylene]-6-methoxycarbonyl-2-indolinone benzoate; in crystalline Form III;
3-Z-[1-(4-(N-((4-methyl-piperazin-1-yl)-methylcarbonyl)-N-methyl-amino)-anilino)-1-phenyl-methylene]-6-methoxycarbonyl-2-indolinone mandelate in crystalline monohydrated Form I;
3-Z-[1-(4-(N-((4-methyl-piperazin-1-yl)-methylcarbonyl)-N-methyl-amino)-anilino)-1-phenyl-methylene]-6-methoxycarbonyl-2-indolinone saccharinate in crystalline hydrated Form II;
3-Z-[1-(4-(N-((4-methyl-piperazin-1-yl)-methylcarbonyl)-N-methyl-amino)-anilino)-1-phenyl-methylene]-6-methoxycarbonyl-2-indolinone saccharinate in crystalline Form III;
3-Z-[1-(4-(N-((4-methyl-piperazin-1-yl)-methylcarbonyl)-N-methyl-amino)-anilino)-1-phenyl-methylene]-6-methoxycarbonyl-2-indolinone saccharinate in crystalline hydrated Form V;
3-Z-[1-(4-(N-((4-methyl-piperazin-1-yl)-methylcarbonyl)-N-methyl-amino)-anilino)-1-phenyl-methylene]-6-methoxycarbonyl-2-indolinone salicylate in crystalline Form I;
3-Z-[1-(4-(N-((4-methyl-piperazin-1-yl)-methylcarbonyl)-N-methyl-amino)-anilino)-1-phenyl-methylene]-6-methoxycarbonyl-2-indolinone salicylate in crystalline anhydrous Form II;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}L-aspartate in crystalline hydrated Form I;
Bis{3-Z-[1-(4-(N-((4-methyl-piperazin-1-yl)-methylcarbonyl)-N-methyl-amino)-anilino)-1-phenyl-methylene]-6-methoxycarbonyl-2-indolinone}L-aspartate in crystalline Form II;
3-Z-[1-(4-(N-((4-methyl-piperazin-1-yl)-methylcarbonyl)-N-methyl-amino)-anilino)-1-phenyl-methylene]-6-methoxycarbonyl-2-indolinone xinafoate in crystalline monohydrated Form I;
3-Z-[1-(4-(N-((4-methyl-piperazin-1-yl)-methylcarbonyl)-N-methyl-amino)-anilino)-1-phenyl-methylene]-6-methoxycarbonyl-2-indolinone ascorbate in crystalline Form I;
3-Z-[1-(4-(N-((4-methyl-piperazin-1-yl)-methylcarbonyl)-N-methyl-amino)-anilino)-1-phenyl-methylene]-6-methoxycarbonyl-2-indolinone ascorbate in crystalline Form III;
3-Z-[1-(4-(N-((4-methyl-piperazin-1-yl)-methylcarbonyl)-N-methyl-amino)-anilino)-1-phenyl-methylene]-6-methoxycarbonyl-2-indolinone ascorbate in crystalline Form IV;
3-Z-[1-(4-(N-((4-methyl-piperazin-1-yl)-methylcarbonyl)-N-methyl-amino)-anilino)-1-phenyl-methylene]-6-methoxycarbonyl-2-indolinone ascorbate in crystalline Form VI; and
3-Z-[1-(4-(N-((4-methyl-piperazin-1-yl)-methylcarbonyl)-N-methyl-amino)-anilino)-1-phenyl-methylene]-6-methoxycarbonyl-2-indolinone ascorbate in crystalline Form VII.

4. Salt or crystalline salt form in accordance with any one of claims 1 to 3 for its use as medicament.

5. Pharmaceutical composition comprising a salt or a crystalline salt form in accordance with any one of claims 1 to 3, together with one or more inert carriers or diluents.
